# EUROPEAN PATENT SPECIFICATION

(11) **EP 3 494 908 B1**
(45) Date of publication and mention of the grant of the patent: **01.09.2021**
(21) Application number: 19154625.8
(22) Date of filing: 13.03.2015
(51) Int. Cl.: A61B 17/28

(54) **SURGICAL CLAMP JAW**
CHIRURGISCHE KLEMMBACKE
MÂCHOIRE DE PINCE CHIRURGICALE

(30) Priority: 13.03.2014 US 201414207813; 13.03.2014 US 201414207839
(43) Date of publication of application: 12.06.2019
(62) Divisional of application: 15761931.3
(73) Proprietor: LSI Solutions, Inc., Victor, NY 14564 (US)
(72) Inventor: SAUER, Jude S., Pittsford, NY 14534 (US)
(74) Representative: Flügel Preissner Schober Seidel

(56) References cited:
- US-A1- 2006 028 041
- US-A1- 2011 039 967
- US-A1- 2011 288 579

## Description

### FIELD

The claimed invention relates to clamping devices, and more specifically to a surgical clamping device.

### BACKGROUND

The ability of cardiac surgeons to successfully treat an ever-increasing number of heart conditions is well-documented. From relatively crude attempts by surgeons to repair stab wounds to the heart at start of the 20^{th} century, to exploratory attempts in the first half of the 1900's to open or repair heart valves before reliable cardio-pulmonary bypass (CPB) became available, to the steady stream of advances in cardiology in the second half of the 20^{th} century, including CPB improvements and cold blood cardioplegia techniques to enable increased operating time while minimizing damage to the heart, prosthetic heart valve development for mitral and aortic valve replacement, coronary artery bypass grafting, and a host of other cardiac procedures, open heart surgery continues to improve at an impressive rate. As the 21^{st} century is well underway, cardiac surgery continues to improve, with a focus on less invasive heart surgery.

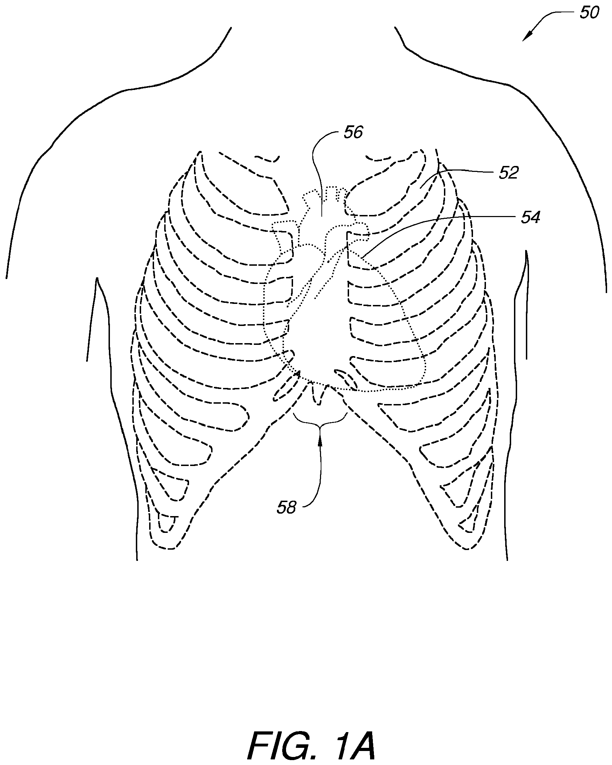

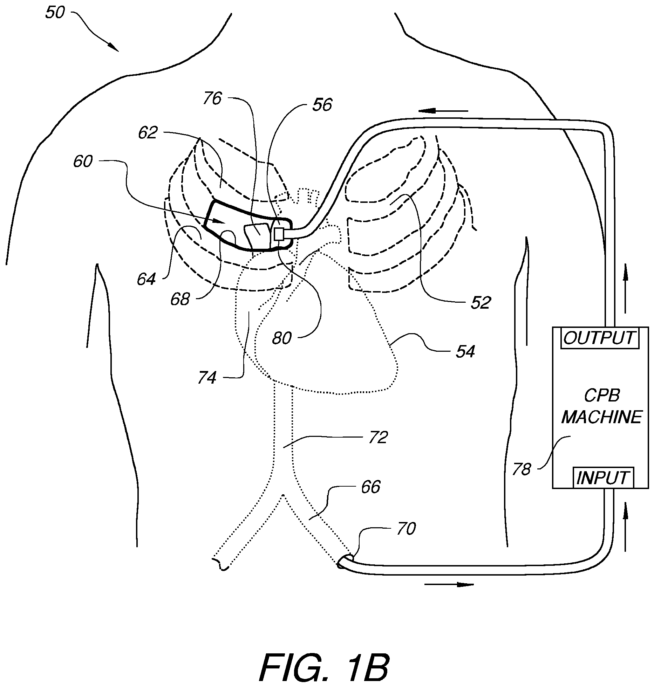

A variety of technologies, knowledge, and surgical skills are relied upon to enable less invasive cardiac surgery. As an example, consider one method of aortic valve replacement and the logistical situation presented by such a surgery as highlighted in FIGS. 1A and 1B. FIG. 1A schematically illustrates a human thorax 50 outlined in a solid line with ribcage 52 approximated with the broken lines. A heart 54 and some of the veins and arteries leading to and from the heart 54 are represented by the dotted lines. The heart 54, and in particular, the aorta 56 are generally well protected by the ribcage 52. Before recent advances in cardiac surgery, it was frequently necessary to "crack" the sternum 58 and spread the left and right halves of the ribcage 52 following a sternotomy (incision which can run twenty centimeters (cm) or more down a patient's chest over the sternum 58). However, while providing excellent access to the heart 54 it is preferable to avoid a sternotomy and the long recovery times and high levels of pain associated with such invasive surgery.

As an alternative, surgeons are often able to use a thoracotomy (preferably an incision between the ribs) as an access point to operate on the heart. FIG. 1B schematically illustrates a thoracotomy in the context of this aortic valve replacement example. A minimally invasive incision 60 is made in the right second intercostal space (between the second and third ribs 62, 64) while simultaneously gaining percutaneous access to a femoral vein 66. Muscle is dissected from the ribs 62, 64 and retractors (not shown) are placed to spread the incision 60 and the ribs 62, 64 to create a minimally invasive opening 68. With access available through a less invasive opening 68, the pericardium is incised over the aorta 56. Stay sutures (not shown) can be placed in tissues and pulled back to increase access to the aorta 56.

A venous cannula 70 is prepared and inserted after dilation of the percutaneous incision in the femoral vein 66. A guide wire can be placed into the venous cannula 70 and threaded through the femoral vein 66, through the inferior vena cava 72, into the right atrium 74 of the heart 54, and into the superior vena cava 76. A series of dilators (not shown) are used to widen the guide wire tract to the venous cannula 70. The venous cannula 70 is attached to the input side of a CPB machine 78, providing a path for the bypass machine 78 to grab deoxygenated blood that has returned from the body to the heart 54. An aortic cannula 80 is also placed and attached to the output side of the bypass machine 78, providing a place for reoxygenated blood (supplied by the bypass machine 78) to be returned to the body.

The superior vena cava 76 is dissected away from the aorta 56, and an aortic cross clamp (not shown) is introduced on the heart side of the aortic cannula 80, but away from the aortic valve. The aortic cross clamp seals the aorta 56 so that the CPB machine 78 can begin circulating oxygenated blood to the body without leakage back through the heart 54. The heart 54 is stopped, for example, by medication and/or lowering the temperature of the heart, and a transverse aortotomy is created to expose the aortic valve. The defective aortic valve is then cut out and care is taken to remove any debris, such as calcium or plaque deposits, which may have accumulated on the valve and come loose during the valve removal.

A replacement aortic valve (either mechanical, synthetic, or donor tissue) is then seated and sewed into place. The aortotomy is then closed. The aortic cross clamp is removed, the heart 54 is restarted, and the cannula connection points 70, 80 are removed to disconnect the patient from the CPB machine 78. Finally, the remaining open incisions are closed.

While the minimally invasive nature of the incisions can result in shortened patient recovery times, the demands on a surgeon during such a procedure can be high considering that the surgeon has a very small incision window 68 within which the operation must take place. Space is at a premium, and the surgeon must find a way to manage tubing for the aortic cannula and associated tubing for the CPB machine, the aortic cross-clamp, pull-back sutures, closure sutures for the cannula incision, and any of the necessary scalpels, manipulators, suture needles, and suction devices so that there is still room to install the replacement valve through the opening 68 in the ribs.

Recent efforts have been directed towards the aortic cross clamp, in particular, in order to minimize the amount of space which is taken up by the clamp. For example, U.S. Patent No. 8,303,611 by Danitz discloses a clamping device where the clamp is coupled to a control handle by a flexible shaft. The Danitz flexible shaft also has a telescoping rigid element which temporarily extends from the handle to cover the shaft so that the surgeon has solid control over the clamp when placing it. The rigid cover is then drawn back into the handle, and the flexible shaft can be manipulated during the surgery to less obstructive locations as desired. While the flexibility of the shaft does give a surgeon more options for placement, the shaft still has to be managed during surgery, and any movement of the Danitz shaft can still be transferred to the clamp, increasing the possibility that the clamp might be prematurely jostled loose. Additionally, Danitz's flexible mechanical linkages may not provide sufficient rigidity or holding force.

In an effort to minimize interference from an endoscopic clamp shaft, the clamping device disclosed in U.S. Patent No. 5,921,996 by Sherman has a clamp with jaws that may pivot relative to each other when a spring in the jaw is disengaged by a control on an applicator handle. When the Sherman clamp is attached to the applicator handle, the clamp jaws can be tightened in place. The spring is re-engaged with the jaws, holding them together, and the applicator handle is removed from the clamp. While removal of Sherman's applicator handle from the clamp provides increased access through a minimally invasive access site once the handle is out of the picture, Sherman's handle must be reattached to the clamp in order to open and remove the clamp. Given the keyed nature of Sherman's coupling between the clamp and the applicator handle, reattachment of the applicator handle may be difficult in hard to reach places, especially since the orientation of the clamp may have changed during the surgery as tissue and organs are moved around. There is also the concern that the clamp may be prematurely jostled loose when the clamp is first applied and the applicator handle is removed since the mechanism to disengage the spring might inadvertently be pressed on the handle as the applicator is withdrawn.

US 2011/288579 A1 discloses a manipulator having two gripping jaw which are resilient, while exhibiting two different spring constants depending on the degree of deflection of each gripping jaw. In one embodiment, the two different spring constants are achieved by providing two spring parts which are basically formed by gaps and buffer parts having different material thicknesses. Due to the different thicknesses of the buffer parts, the jaws are deflected upon interaction with an object by firstly bending the first spring part due to its reduced thickness of the buffer part and, thus, the reduced strength. Upon closing the gap, the second spring part provides an increased stiffness.

US 2011/0039967 A1 discloses a microgripper that utilizes a pair of shape memory polymer actuators to open and close the jaws of gripping members according to the temperature the actuators are exposed to.

If an aortic cross clamp inappropriately releases or slips off the aorta, visualization and hemodynamic control can be lost and the patient can exsanguinate. Therefore, there is first and foremost a need for surgical clamp devices that have a reliable and strong clamping force. There is also a need for such surgical clamping devices to be compatible with less invasive cardiac surgical procedures such that surgeons can easily operate and manipulate the clamps through small access sites while preferably creating little to no reduction in the surgical access area. There is further a need for such surgical clamps to be easily releasable and removable without having to realign and reattach removal devices.

### SUMMARY

The objective is solved by the surgical clamp jaw according to claim 1.

According to the invention, a surgical clamp jaw, comprises an inner profile and a deflection control profile opposite the inner profile. The deflection control profile comprises one or more sets of corresponding abutment surfaces, wherein the abutment surfaces in at least one of the one or more sets of corresponding abutment surfaces are not contacting each other when the inner profile is in an unclamped position; and the abutment surfaces in the at least one of the one or more sets of corresponding abutment surfaces are in contact with each other when the inner profile is in a clamped position. The corresponding abutment surfaces in the at least one of the one or more sets of corresponding abutment surfaces, comprise a first abutment surface having a first interlocking feature and a second abutment surface having a second interlocking feature; wherein the first and second interlocking features are not in contact when the inner profile is in the unclamped position; and wherein the first and second interlocking features are configured to interlock when the inner profile is in the clamped position.

In a preferred embodiment, the abutment surfaces in at least one of the one or more sets of corresponding abutment surfaces are not contacting each other when the inner profile is in an unclamped position, and the abutment surfaces in at least one of the one or more sets of corresponding abutment surfaces are in contact with each other when the inner profile is in a clamped position.

In a preferred embodiment, the inner profile comprises a first substantially concave profile in the unclamped position, and the inner profile comprises a second substantially concave profile in the clamped position.

In a preferred embodiment, the inner profile comprises a substantially concave profile in the unclamped position, and the inner profile comprises a substantially flat profile in the clamped position.

In a preferred embodiment, the inner profile comprises a substantially concave profile in the unclamped position, and the inner profile comprises a substantially convex profile in the clamped position.

In a preferred embodiment, the surgical clamp jaw defines one or more flexion assistance voids.

In a preferred embodiment, the one or more flexion assistance voids have a sectional shape in a plane substantially perpendicular to the inner profile, the sectional shape selected from the group consisting of substantially triangular, substantially circular, substantially rectangular, and substantially oval.

In a preferred embodiment, at least one of the one or more flexion assistance voids is in contact with a gap between one of the one or more sets of corresponding abutment surfaces.

In a preferred embodiment, the surgical clamp jaw further comprises a pivot point.

In a preferred embodiment, the surgical clamp jaw further comprises an actuator configured to rotate the inner profile of the surgical clamp jaw around the pivot point.

In a preferred embodiment, the actuator is selected from the group consisting of a lever, an arm, a gear, a pulley, and a motor.

In a preferred embodiment, the inner profile comprises a smooth surface.

In a preferred embodiment, the surgical clamp jaw further comprises a shod.

### BRIEF DESCRIPTION OF THE DRAWINGS

FIG. 1A schematically illustrates the relative locations of the heart and ribcage in a human thorax.
FIG. 1B schematically illustrates a cardiac surgical procedure performed through an intercostal space.
FIG. 2 illustrates one embodiment of a surgical clamping device having a locking suture.
FIG. 3 illustrates one embodiment of a surgical clamping device having a locking suture and an opening suture.
FIG. 4 illustrates one embodiment of a surgical clamping device having locking, opening, and closing sutures.
FIG. 5 illustrates one embodiment of a surgical clamping device having a removably coupled introducer shaft.
FIG. 6 illustrates one embodiment of a surgical clamping device having multiple introducer connection points for a removably coupled introducer shaft.
FIG. 7 illustrates a surgical clamping device having another embodiment of clamping jaws.
FIGS. 8A and 8B are perspective views of one embodiment of a surgical clamping device having an articulator.
FIG. 9 is an exploded perspective view of the surgical clamping embodiment of FIGS. 8A and 8B.
FIGS. 10A-10E schematically illustrate one embodiment of a process of introducing a surgical clamping device through a surgical access point using an introducer shaft, positioning first and second clamp jaws around an internal structure, and applying a mechanical knot to a locking suture coupled to first and second locking suture guides to lock the clamp jaws in a clamped position around the internal structure. The introducer shaft is also removed in FIG. 9E.
FIG. 11 illustrates one embodiment of an aortic cross clamp locked in a clamped position by a mechanical knot.
FIG. 12 is a top view of a surgical clamping device having one embodiment of suture retainers.
FIG. 13 is a partially exploded perspective view of the surgical clamping device of FIG. 12.
FIG. 14 is a perspective view of another embodiment of a surgical clamping device having suture retainers.
FIGS. 15A and 15B are perspective and top views, respectively, of a surgical clamping device having suture retainers and locked in a clamped position by a mechanical knot.
FIGS. 16-18F illustrate different embodiments of surgical clamping devices locked in a clamped position by a mechanical knot.
FIGS. 19A-19B illustrate one embodiment of a method for clamping a structure during surgery.
FIG. 20A is a top view of one embodiment of surgical clamp jaws pivotably held by a housing.
FIG. 20B is a side view of the embodied surgical clamp jaws of FIG. 20A.
FIG. 20C is an exploded perspective view of the embodied surgical clamp jaws of FIG. 20A.
FIG. 21A illustrates one embodiment of a surgical clamp jaw in an unclamped position.
FIG. 21B is an enlarged view of a portion of the surgical clamp jaw of FIG. 21A, featuring one embodiment of corresponding abutment surfaces and one embodiment of flexion assistance voids.
FIG. 21C illustrates the embodied surgical clamp jaw of FIG. 21A in a clamped position featuring a substantially flat inner profile.
FIG. 22A illustrates another embodiment of a surgical clamp jaw in an unclamped position.
FIG. 22B is an enlarged view of a portion of the surgical clamp jaw of FIG. 22A, featuring another embodiment of corresponding abutment surfaces and one embodiment of flexion assistance voids.
FIG. 22C illustrates the embodied surgical clamp jaw of FIG. 22A in a clamped position featuring a substantially concave inner profile.
FIG. 23A illustrates a further embodiment of a surgical clamp jaw in an unclamped position.
FIG. 23B illustrates the embodied surgical clamp jaw of FIG. 23A in a clamped position featuring a substantially convex inner profile.
FIGS. 24 and 25 illustrate other embodiments of surgical clamp jaws having examples of different flexion assistance void distribution.
FIGS. 26-28 illustrate further embodiments of surgical clamp jaws featuring examples of different flexion assistance void shapes.
FIG. 29A illustrates another embodiment of a surgical clamp jaw where the sets of corresponding abutment surfaces are not contiguous with the flexion assistance voids.
FIG. 29B is an enlarged view of a portion of the surgical clamp jaw of FIG. 29A.
FIG. 30A illustrates an embodiment of the surgical clamp jaw of FIG. 21A also having an example of an integral gripping surface on the inner profile.
FIG. 30B is an enlarged view of a portion of the surgical clamp jaw of FIG. 30A.
FIG. 31A illustrates a surgical clamp jaw having interlocking features on corresponding abutment surfaces according to an embodiment of the invention.

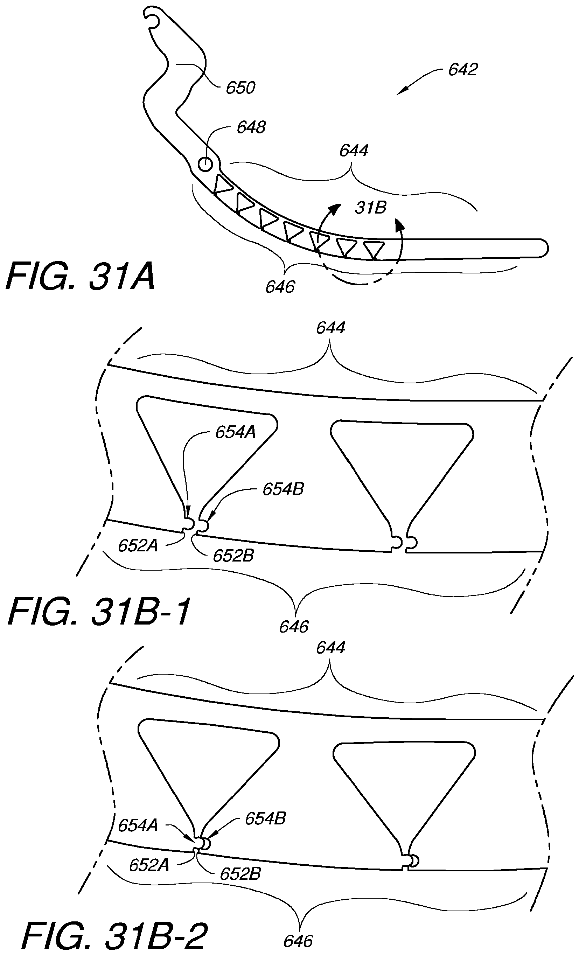

FIGS. 31B-1 and 31B-2 are enlargements of alternate examples of the interlocking features for a surgical clamp jaw based on the embodiment of FIG. 31A, the embodiment of FIG. 31B-1 forms part of the invention and the example of FIG 31B-2 does not form part of the invention.

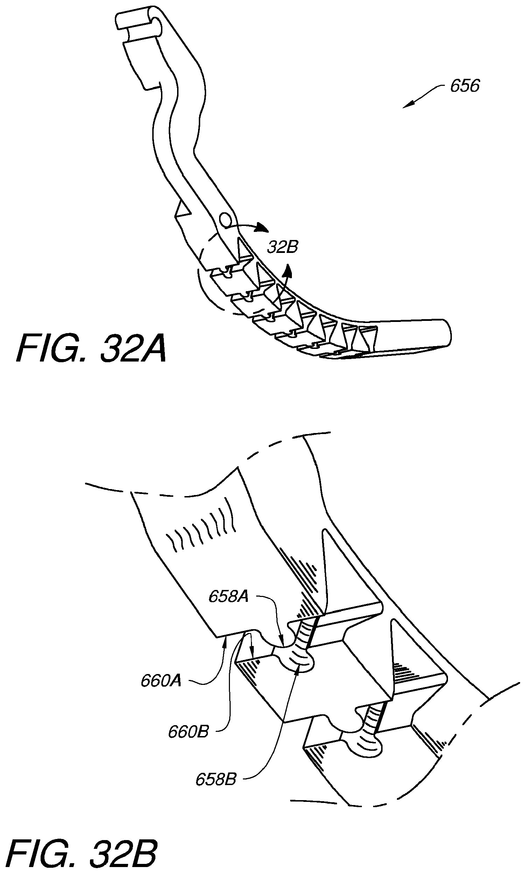

FIG. 32A illustrates a further a surgical clamp jaw having interlocking features on corresponding abutment surfaces according to an embodiment of the invention.
FIG. 32B is an enlarged view of a portion of the surgical clamp jaw of FIG. 32A.

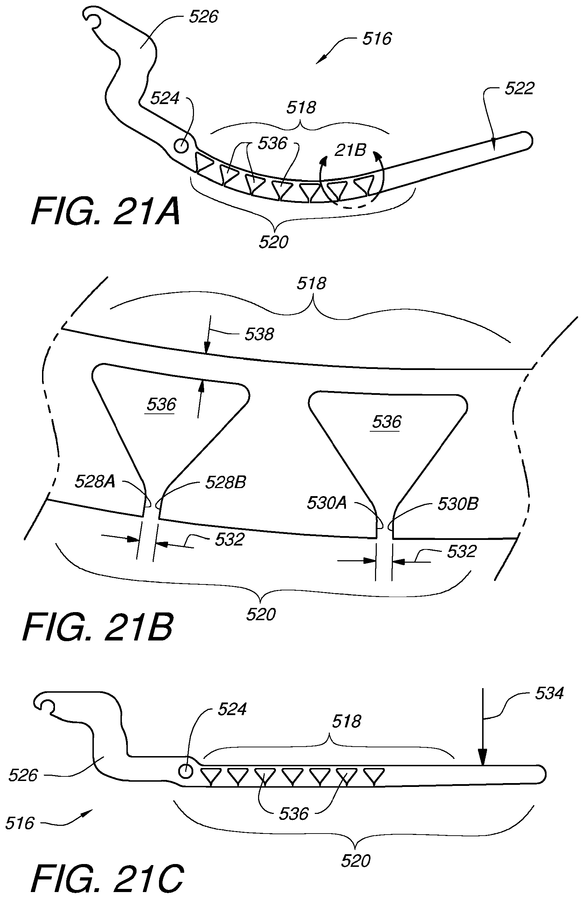

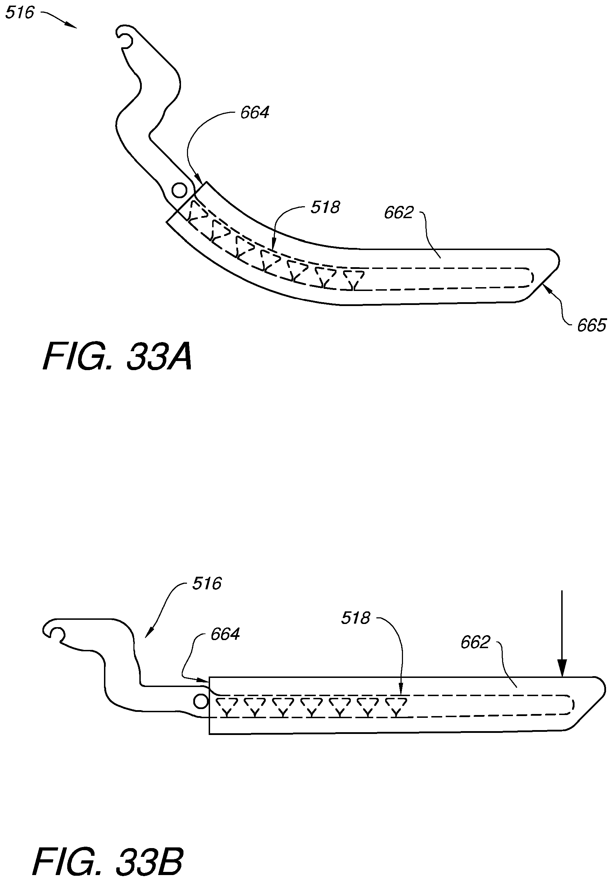

FIG. 33A illustrates the embodied surgical clamp jaw of FIG. 21A, in an unclamped position, with one embodiment of a shod.
FIG. 33B illustrates the embodied surgical clamp jaw of FIG. 33A in a clamped position.

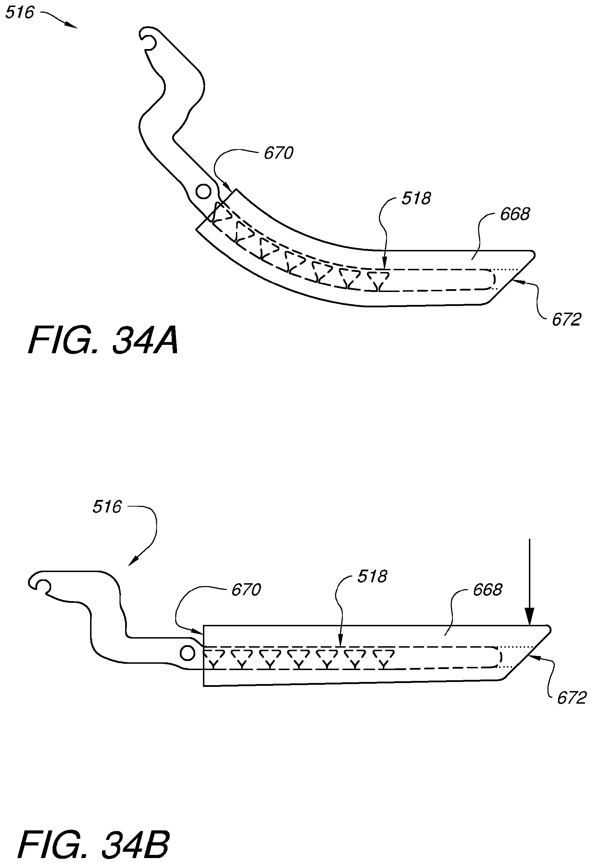

FIG. 34A illustrates the embodied surgical clamp jaw of FIG. 21A, in an unclamped position, with another embodiment of a shod.
FIG. 34B illustrates the embodied surgical clamp jaw of FIG. 34A in a clamped position.

It will be appreciated that for purposes of clarity and where deemed appropriate, reference numerals have been repeated in the figures to indicate corresponding features, and that the various elements in the drawings have not necessarily been drawn to scale in order to better show the features.

### DETAILED DESCRIPTION

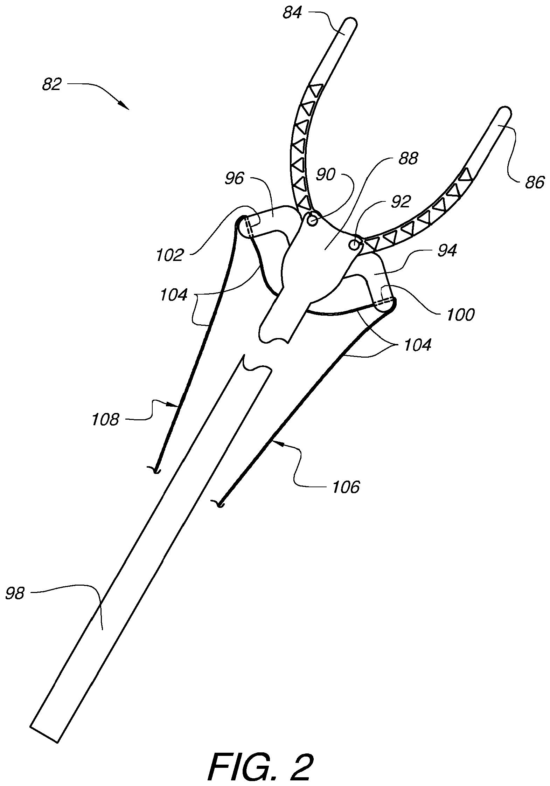

FIG. 2 illustrates one embodiment of a surgical clamping device 82. The device 82 has surgical clamp jaws 84, 86 pivotably held by a housing 88. The clamp jaw 84 pivots around pivot point 90, while clamp jaw 86 pivots around pivot point 92. A variety of clamp jaw types and shapes are compatible with the embodiments disclosed herein. The features of the clamp jaw illustrated in FIG. 2, and in many of the following embodiments, will be discussed in more detail later in the specification.

Each clamp jaw 84, 86 may be coupled to an actuator 94, 96 configured to rotate the respective surgical clamp jaw 84, 86 around its respective pivot point 90, 92. In this embodiment, the actuators 94, 96 are control arms. In the orientation of FIG. 2, the actuator 94 can be rotated in a clockwise arc around pivot point 90 to move surgical clamp jaw 84 in a similar direction towards surgical clamp jaw 86. Likewise, the actuator 96 can be rotated in a counterclockwise arc around pivot point 92 to move surgical clamp jaw 86 in a similar direction towards surgical clamp jaw 84.

An introducer shaft 98 is coupled to the housing 88. The housing 88 is pivotably coupled to the clamp jaws 84, 86 via the pivot points 90, 92. Since the introducer shaft 98 is coupled to the housing 88, the introducer shaft 98 is therefore also pivotably coupled to the clamp jaws 84, 86 by association. In some embodiments, the introducer shaft 98 could be directly coupled to at least one of the clamp jaws 84, 86 without the need for a separate housing 88. The introducer shaft 98 can be manipulated by a surgeon to guide the clamp jaws 84, 86 through a surgical entry point and help to position the jaws 84, 86 as desired. Each of the actuators 94, 96 has a respective locking suture guide 100, 102. In this embodiment, the locking suture guides 100, 102 are channels formed in the actuator arms 94, 96. A locking suture 104 is threaded through one locking suture guide 100, past the housing 88, and through the other locking suture guide 102. The ends 106, 108 of the locking suture 100 (shown shortened for convenience in this view) are available to the surgeon for locking the clamp jaws 84, 86 in a clamped position with a knot, such as a mechanical knot, as will be described in more detail later in this specification. Before a knot is formed, as the ends 106, 108 of the locking suture 100 are pulled away from the clamp jaws 84, 86, the actuators 94, 96 will be pulled towards each other, causing the clamp jaws 84, 86 to close. Therefore, in some embodiments, the locking suture 100 can also be considered as a closing suture. This closing functionality can be useful to a surgeon because the suture ends 106, 108 can be manipulated remotely to help position the clamp jaws 84, 86. Additionally, although a single locking suture 104 is used in this embodiment, other embodiments can have multiple locking sutures. For example, as one alternative, a first locking suture could be tied off or otherwise connected to a first of the locking suture guides, while a second, separate locking suture could be tied off or otherwise connected to a second of the locking suture guides. The two ends 106, 108 will still operate in a similar fashion, even though the ends would not be part of the same continuous suture in such an embodiment. It should be understood that the term "suture", as used herein, is intended to cover any thread, cable, wire, filament, strand, line, yarn, gut, or similar structure, whether natural and/or synthetic, in monofilament, composite filament, or multifilament form (whether braided, woven, twisted, or otherwise held together), as well as equivalents, substitutions, combinations, and pluralities thereof for such materials and structures.

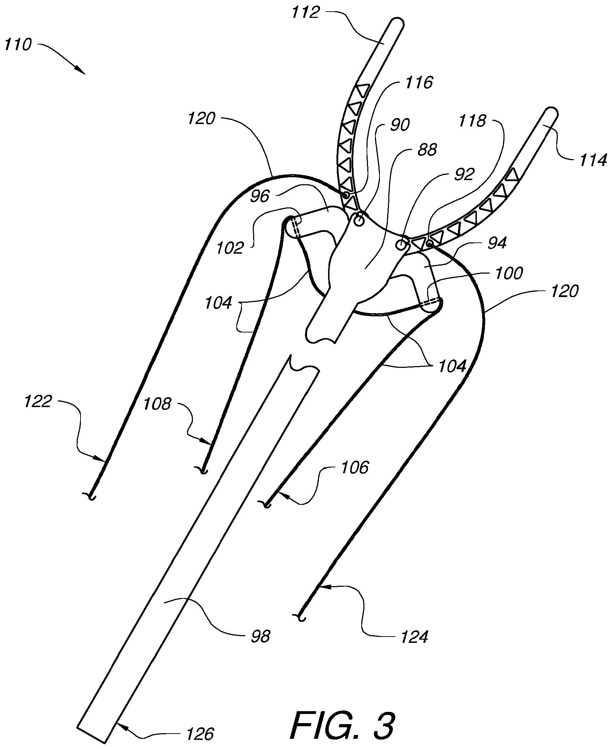

FIG. 3 illustrates another embodiment of a surgical clamping device 110 which is similar to the clamping device of FIG. 2, discussed previously. The clamp jaws 112, 114 of the device 110 in FIG. 3, however, also each have an opening connection point 116, 118, respectively. An opening suture 120 is threaded through one opening connection point 116, past the housing (this portion of the path is not shown), and through the other opening connection point 118. The ends 122, 124 of the opening suture 120 (shown shortened for convenience in this view) are available to the surgeon for opening the clamp jaws 112, 114. For example, if the ends 122, 124 of the opening suture 120 are pulled away from the clamp jaws 112, 114 in a direction generally towards the proximal end 126 of the introducer shaft 98, the clamp jaws 112, 114 will be pulled open. This opening functionality can be useful to a surgeon because the suture ends 122, 124 (of the opening suture 120) can be manipulated remotely to help position the clamp jaws 112, 114. Like the previous embodiment, the ends 106, 108 (of the locking suture 104) can also be used to help close the jaws 112, 114 as previously described.

Although a single opening suture 120 is used in this embodiment, other embodiments can have multiple opening sutures. For example, as one alternative, a first opening suture could be tied off or otherwise connected to a first of the opening connection points 116, while a second, separate opening suture could be tied off or otherwise connected to a second of the opening suture connection points 118. The two ends 122, 124 will still operate in a similar fashion, even though the ends would not be part of the same continuous suture in such an embodiment.

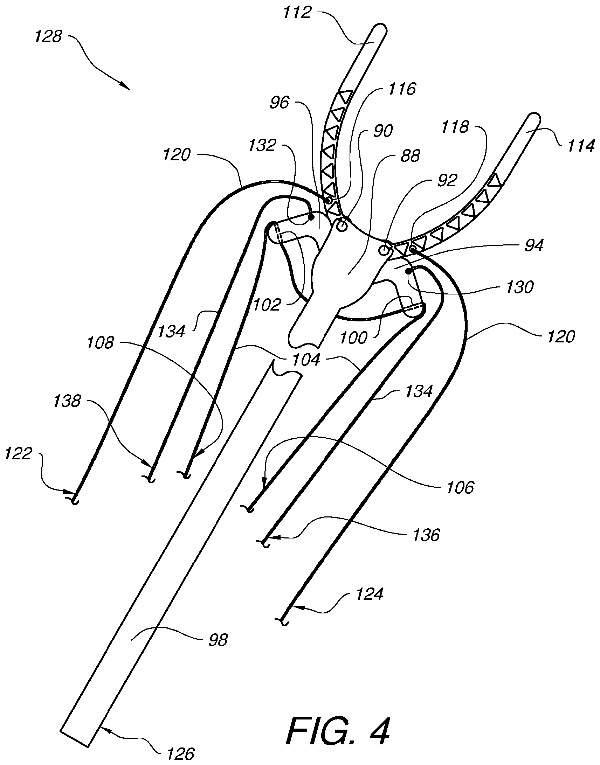

FIG. 4 illustrates another embodiment of a surgical clamping device 128 which is similar to the clamping device of FIG. 3, discussed previously. The actuator arms 94, 96 of the device 128 in FIG. 4, however, also each have a closing connection point 130, 132, respectively. A closing suture 134 is threaded through one closing connection point 130, past the housing (this portion of the path is not shown), and through the other closing connection point 132. The ends 136, 138 of the closing suture 134 (shown shortened for convenience in this view) are available to the surgeon for closing the clamp jaws 112, 114. For example, if the ends 136, 138 of the closing suture 134 are pulled away from the clamp jaws 112, 114 in a direction generally towards the proximal end 126 of the introducer shaft 98, the clamp jaws 112, 114 will be pulled closed. This closing functionality can be useful to a surgeon because the suture ends 136, 138 (of the closing suture 134) can be manipulated remotely to help position the clamp jaws 112, 114. Like the previous embodiment, the ends 122, 124 (of the opening suture 120) can also be used to help open the jaws 112, 114 as previously described. Embodiments such as the one shown in FIG. 4 do not require the locking suture 104 to double as a closing suture since a separate closing suture 134 is provided.

Although a single closing suture 134 is used in this embodiment, other embodiments can have multiple closing sutures. For example, as one alternative, a first closing suture could be tied off or otherwise connected to a first of the closing connection points 130, while a second, separate closing suture could be tied off or otherwise connected to a second of the closing connection points 132. The two ends 136, 138 will still operate in a similar fashion, even though the ends would not be part of the same continuous suture in such an embodiment.

Although the ends of the various locking, opening, and closing sutures 104, 120, 134 are shown hanging loose in the embodiments above, other embodiments could include one or more suture routing features to help guide any or all of the sutures along the introducer shaft or to help hold the various sutures to keep them organized until the surgeon has need to interact with them.

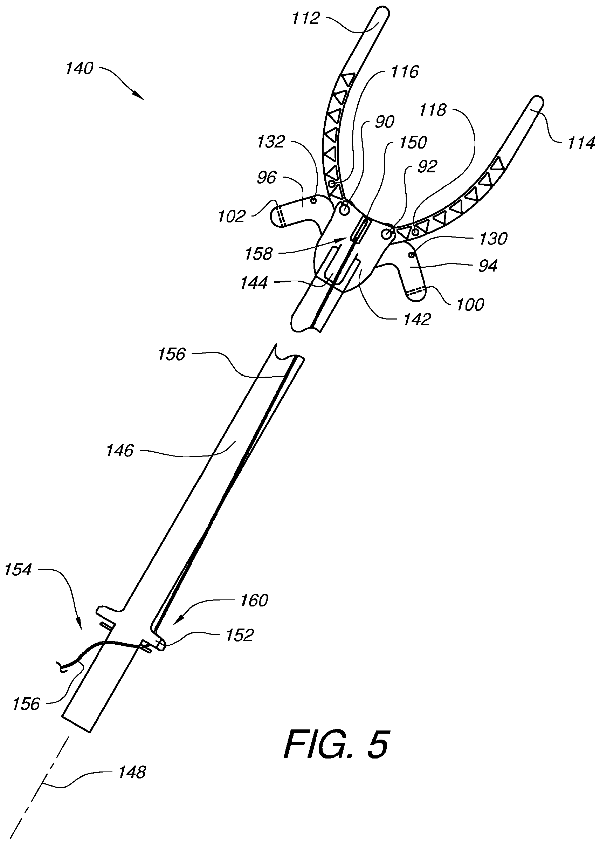

FIG. 5 illustrates another embodiment of a surgical clamping device 140, similar to the clamping device of FIG. 4, discussed previously. However, the housing 142, of the clamping device 140 of FIG. 5, has an introducer connection point 144 to which the introducer shaft 146 may be removably coupled. In this example, the introducer connection point 144 slides into corresponding features of the introducer shaft 146 along a direction substantially parallel to a longitudinal axis 148 of the introducer shaft 146.

The surgical clamping device 140 also has a tie-down suture connection point 150 on the housing 142 and at least one cleat 152 coupled to the introducer shaft 146, in this case, near the proximal end 154 of the shaft 146. A tie-down suture 156 couples to the tie-down suture connection point 150 at a first end 158 of the tie-down suture 156 and is removably attached to the cleat 152 at a second end 160 of the tie-down suture 156. In this embodiment, the tie-down suture could be an RD® QUICK LOAD® suture from LSI Solutions, Inc. of Victor, N.Y. (ordering contact information available at www.lsisolutions.com). The RD® QUICK LOAD® suture has a ferrule attached to one end which can be slid into and held by the tie-down suture connection point 150, thereby anchoring the first end 158 of the tie-down suture 156. The second end 154 of the tie-down suture 156 is readily held by the cleat 152.

For embodiments using a tie-down suture 156 as an introducer locking feature, other types of sutures may be coupled to the tie-down suture connection point 150, as will be appreciated by those skilled in the art. Still other embodiments may utilize entirely different introducer locking features to removably couple the introducer shaft 146 to the housing 142. The tie-down suture embodiment, however, provides the advantage that the introducer shaft 146 may be released from the housing 142 by releasing the tie-down suture 156 from the cleat 152 at the proximal end 154 of the device 140. Since this release action takes place on the proximal end 154, the surgeon can make the necessary adjustment outside of a patient's body where the tie-down suture 156 is very easy to reach.

For convenience, the opening suture, closing suture, and locking suture discussed in previous embodiments are not shown in FIG. 5, however it should be understood that the embodiment of FIG. 5 can have such elements included with the device as previously described. The operation of the opening, closing, and locking sutures will be discussed with respect to the tie-down/removal suture 156 later in this specification.

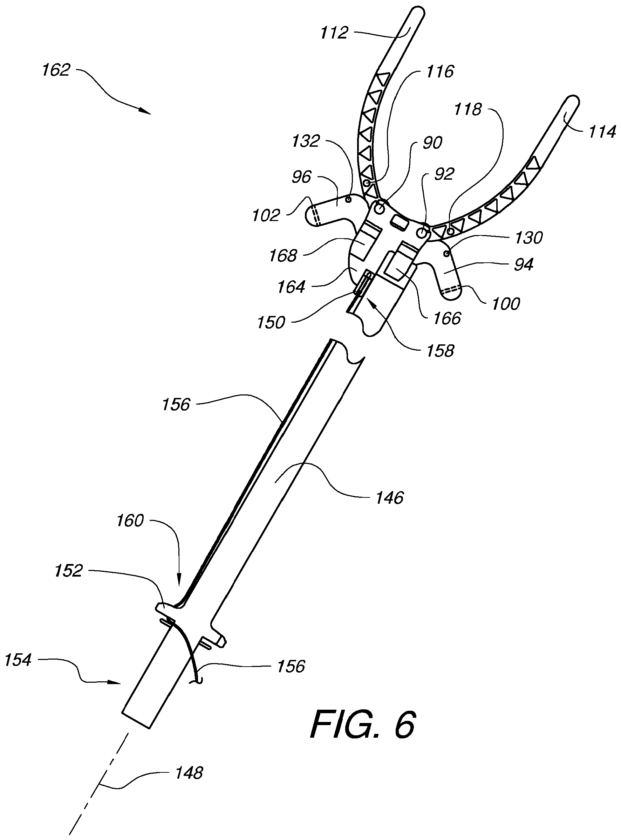

FIG. 6 illustrates another embodiment of a surgical clamping device 162, similar to the clamping device of FIG. 5, discussed previously. However, the housing 164, of the clamping device 162 of FIG. 6, has multiple introducer connection points 166, 168 to which the introducer shaft 146 may be removably coupled. In this example, the introducer connection point 166 is removably coupled to the introducer shaft 146. However, the device 162 could also be set up to be coupled at connection point 168. Since the introducer connection points 166, 168 are located in non-centered positions on the housing 164, the surgeon may advantageously select a connection point 166, 168 which causes the clamp jaws 112, 114 to be centered to one side or the other of the introducer shaft 146, which may assist the surgeon in placing the clamp while minimizing interference from the shaft 146 during placement. In this embodiment, the introducer connection point 166 (and similarly with connection point 168) slides into corresponding features of the introducer shaft 146 along a direction substantially parallel to a longitudinal axis 148 of the introducer shaft 146.

The surgical clamping device 162 also has a tie-down suture connection point 150 on the housing 164 and at least one cleat 152 coupled to the introducer shaft 146, in this case near the proximal end 154 of the shaft 146. A tie-down suture 156 couples to the tie-down suture connection point 150 at a first end 158 of the tie-down suture 156 and is removably attached to the cleat 152 at a second end 160 of the tie-down suture 156. As with the previous embodiment, the tie-down suture 156 could be an RD® QUICK LOAD® suture from LSI Solutions, Inc. of Victor, N.Y. (ordering contact information available at www.lsisolutions.com). The RD® QUICK LOAD® suture has a ferrule attached to one end which can be slid into and held by the tie-down suture connection point 150, thereby anchoring the first end 158 of the tie-down suture 156. The second end 154 of the tie-down suture 156 is readily held by the cleat 152.

For embodiments using a tie-down suture 156 as an introducer locking feature, other types of sutures may be coupled to the tie-down suture connection point 150, as will be appreciated by those skilled in the art. Still other embodiments may utilize entirely different introducer locking features to removably couple the introducer shaft 146 to the housing 164. The tie-down suture method, however, provides the advantage that the introducer shaft 146 may be released from the housing 164 by releasing the tie-down suture 156 from the cleat 152 at the proximal end 154 of the device 162. Since this release action takes place on the proximal end 154, the surgeon can make the necessary adjustment outside of a patient's body where the tie-down suture 156 is very easy to reach.

For convenience, the opening suture, closing suture, and locking suture discussed in previous embodiments are not shown in FIG. 6, however it should be understood that the embodiment of FIG. 6 can have such elements included with the device as previously described. The operation of the opening, closing, and locking sutures will be discussed with respect to the tie-down/removal suture 156 later in this specification.

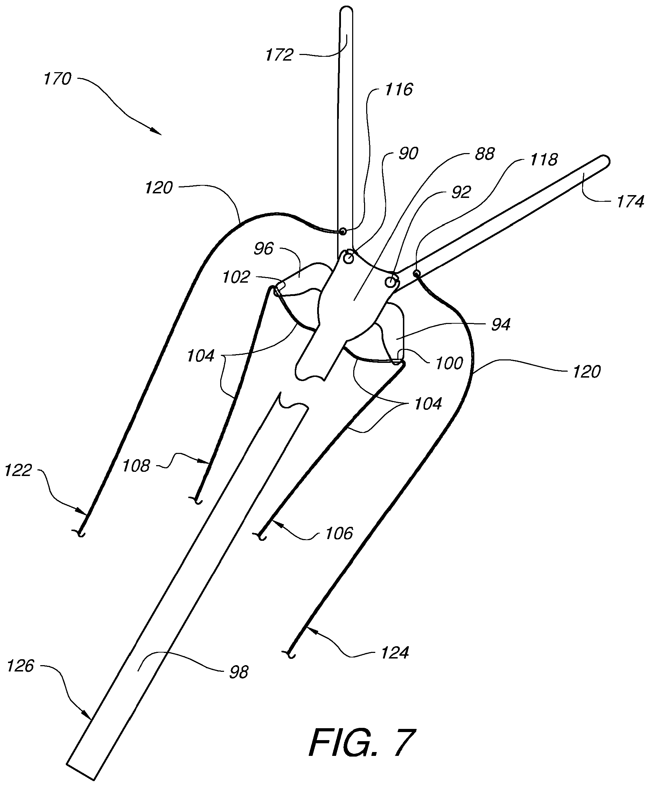

The clamp jaw embodiments 84, 86 of FIG. 2 or the clamp jaw embodiments 112, 114 of FIGS. 3-6 are just some of the clamp jaw configurations which are possible with the claimed invention. As mentioned previously, there are a wide variety of clamp jaws which may be used with embodiments of the surgical devices disclosed herein, and their equivalents. The clamp jaws 84, 86 and 112, 114, illustrated thus far, are partly flexible, and the details of such clamp jaws, as well as variations of such jaws will be discussed further on in this specification. However, non-flexible clamp jaws may also be used, as is illustrated with the embodiment of FIG. 7.

FIG. 7 illustrates another embodiment of a surgical clamping device 170 which is similar to the clamping device of FIG. 3, discussed previously. The clamp jaws 172, 174 of the device 170 in FIG. 7, however, have a different geometry and are substantially flat in unclamped or clamped positions in this example. Like some of the previous embodiments, each clamp jaw 172, 174 has an opening connection point 116, 118, respectively. An opening suture 120 is threaded through one opening connection point 116, past the housing (this portion of the path is not shown), and through the other opening connection point 118. The ends 122, 124 of the opening suture 120 (shown shortened for convenience in this view) are available to the surgeon for opening the clamp jaws 172, 174. For example, if the ends 122, 124 of the opening suture 120 are pulled away from the clamp jaws 172, 174 in a direction generally towards the proximal end 126 of the introducer shaft 98, the clamp jaws 172, 174 will be pulled open. As mentioned previously, this opening functionality can be useful to a surgeon because the suture ends 122, 124 (of the opening suture 120) can be manipulated remotely to help position the clamp jaws 172, 174. Like the previous embodiment, the ends 106, 108 (of the locking suture 104) can also be used to help close the jaws 112, 114 as previously described.

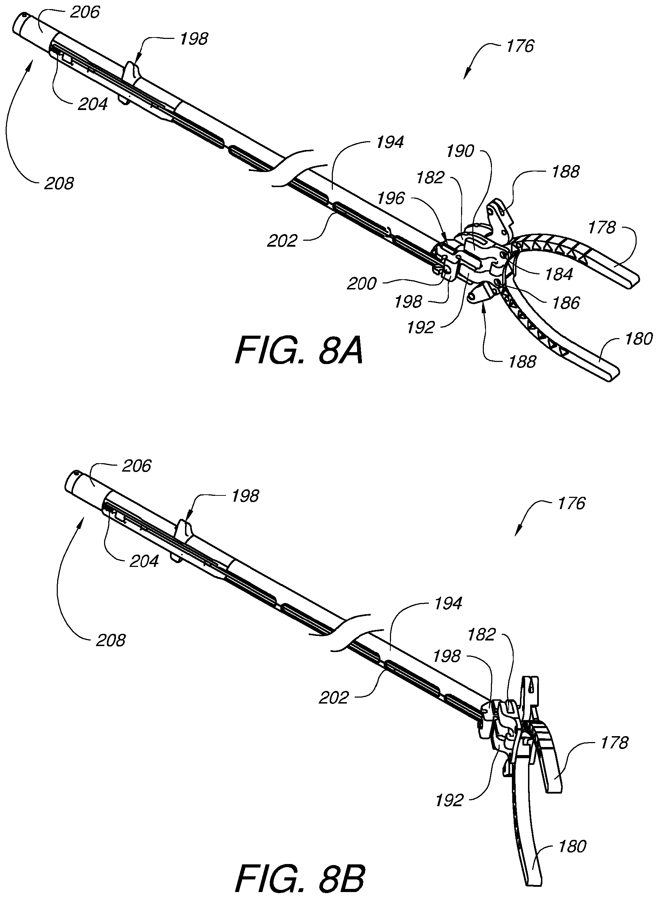

FIGS. 8A and 8B are perspective views of one embodiment of a surgical clamping device 176 having the ability to articulate. The device 176 has first and second clamp jaws 178, 180 which are pivotable in housing 182 around pivot points 184, 186, respectively. Each jaw has a locking suture guide 188 (one of which is visible in the views of FIGS. 8A, 8B). The locking suture guides 188 are configured to receive at least one locking suture for knotting to hold the first and second clamp jaws 178, 180 in a clamped position. For convenience, the locking suture is not shown in these views, however it was discussed previously in this specification.

The housing 182 has multiple introducer connection points 190, 192, similar to embodiments discussed previously. An introducer shaft 194 is removably coupled to introducer connection point 192, however it could instead be removably coupled to introducer connection point 190. The device 176 may have an introducer locking feature for temporarily holding the introducer shaft 194 and the introducer connection point 192 together, as discussed previously. As just one example, an introducer locking feature could include a tie-down suture connection point 196 on the housing 182, at least one cleat 197 coupled to the introducer shaft 194, and a tie-down suture (not shown in this embodiment for convenience), as discussed previously.

The distal end 198 of the introducer shaft 194, where the housing introducer connection point 192 couples to the shaft 194, is pivotable, creating an articulation point whereby the clamp jaws 178, 180 are able to articulate with respect to the introducer shaft 194. The pivoting distal end 198 of the introducer shaft 194 receives a ball 200 coupled to a drive wire 202. The drive wire 202 runs down a channel in the introducer shaft 194 and is coupled to a drive screw 204. A knob 206 is configured to engage drive screw 204 such that knob 206 may be twisted in a first direction to draw drive screw 204 toward the knob 206 (away from the clamp jaws 178, 180) in a direction substantially parallel to the introducer shaft 194. Alternately, the knob 206 may be twisted in a second direction to push drive screw 204 away from the knob 206 (towards the clamp jaws 178, 180) in a direction substantially parallel to the introducer shaft 194. When the drive screw 204, and consequently the drive wire 202 and ball end 200 are pushed toward the clamp jaws 178, 180, the pivoting distal end 198 of the introducer shaft 194 articulates so that the clamp jaws 178, 180 tend to move towards alignment with the introducer shaft 194 (as illustrated in FIG. 8A). Conversely when the drive screw 204, and consequently the drive wire 202 and ball end 200 are pulled away from the clamp jaws 178, 180, the pivoting distal end 198 of the introducer shaft 194 articulates so that the clamp jaws 178, 180 tend to move to a position that is at an angle to the introducer shaft 194 (as illustrated in FIG. 8B). This articulation, controllable on the proximal end 208 of the device, can help a surgeon to manipulate the position of the clamp jaws during a surgery. This may be especially helpful during less invasive surgical procedures where access space is limited.

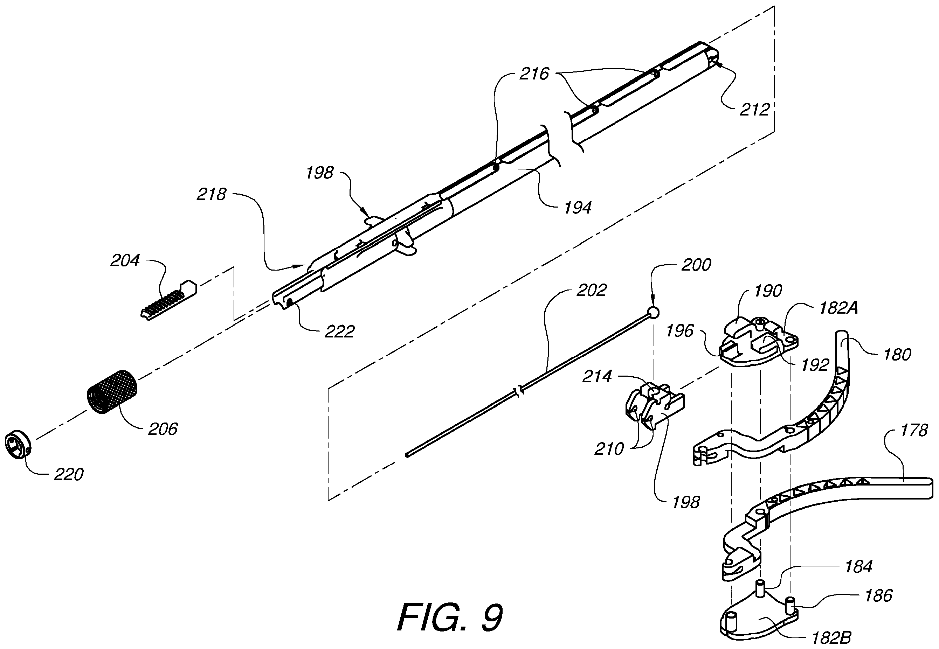

FIG. 9 is an exploded perspective view of the surgical clamping embodiment of FIGS. 8A and 8B which makes it easier to see the various parts of this embodiment. The housing 182 is split into two portions 182A, 182B. The housing 182 supports pivot points 184, 186 on which the jaws 178, 180 pivot. The articulation pivot point 210 of the articulating distal end 198 of the introducer shaft 194 can be seen in FIG. 9. The articulation point 210 corresponds with pivot point 212 on the introducer shaft 194. The ball 200 of the drive wire 202 is received by the recess 214 in the pivoting distal end 198. The drive wire 202 passes through wire supports 216 and is then coupled to the drive screw 204. The drive screw 204 is slidably engaged by the proximal end 218 of the shaft 194, and the knob 206 is threaded onto the drive screw 204. An end cap 220 is then coupled to the end 222 of the shaft 194 to restrain axial movement of the knob 206 while still allowing the knob 206 to rotate. Thus, when the knob 206 is rotated, axial movement is instead imparted to the drive screw 204 and consequently the drive wire 202 as discussed previously.

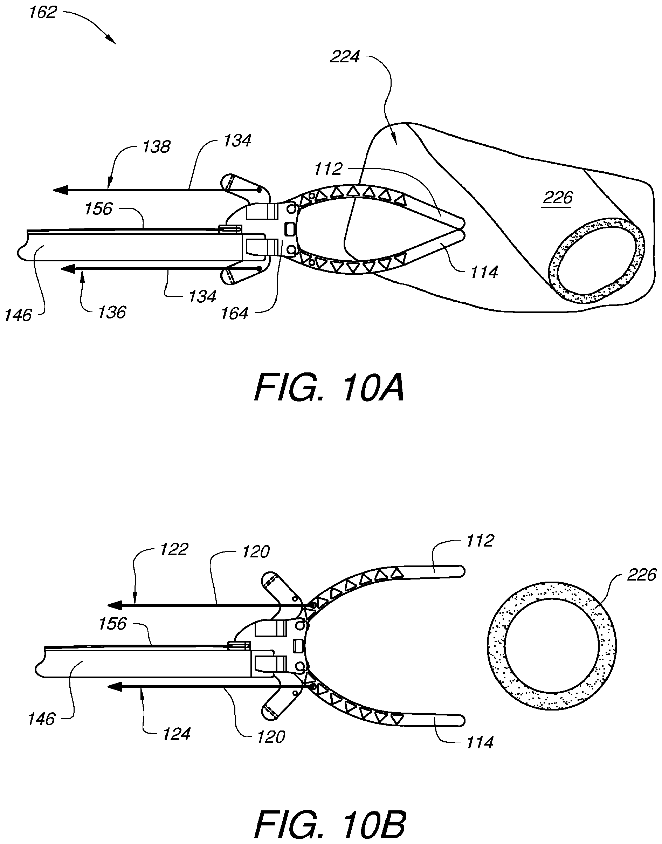

FIG. 10A schematically illustrates one embodiment of a process of introducing a surgical clamping device 162 through a surgical access point 224. This embodiment of a clamping device 162 has been discussed already with regard to FIG. 6. For clarity, FIGS. 10A-10E will only show the suture lines being manipulated or in active use at a given moment. It should be understood, however, that this embodiment includes an opening suture, a closing suture, a locking suture, and a tie down suture, all of which would be in place and coupled to their respective points of the device 162 as described previously. As shown in FIG. 10A, the tie-down suture 156 has the introducer shaft 146 temporarily tied to the housing 164. The ends 136, 138 of the closing suture 134 are pulled back as shown in FIG. 10A, causing the clamp jaws 112, 114 to close. The jaws 112, 114 are pictured in FIG. 10A as partially closed, and an operator could close the jaws more by pulling harder on the closing suture ends 136, 138. The surgical access point 224 in this example is in an intercostal space which provides access to the aorta 226 leading from the heart.

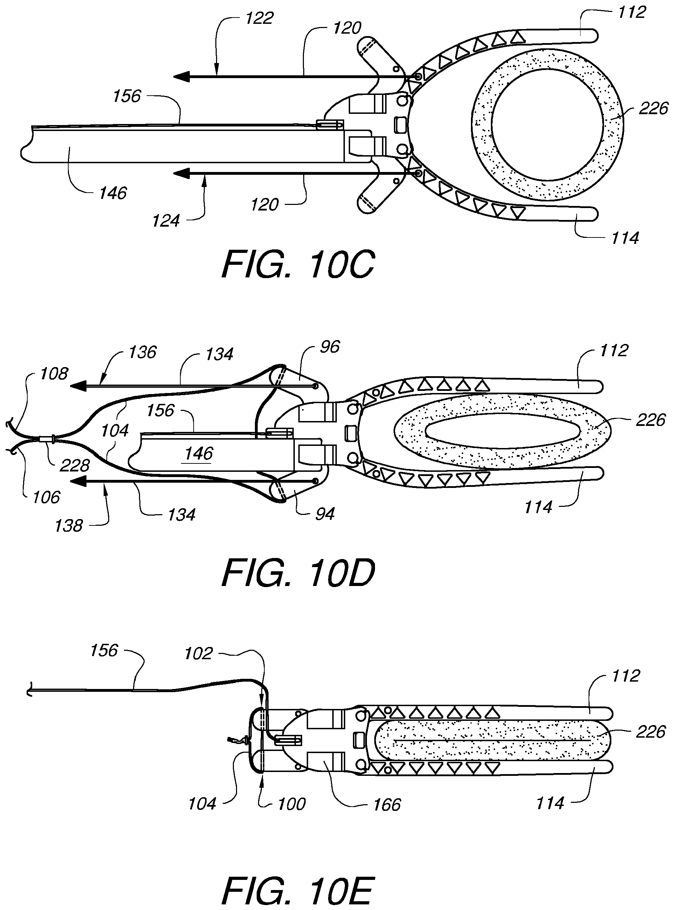

When the jaws 112, 114 are close to the structure 226 which is desired to be clamped the ends 122, 124 of the opening suture 120 may be pulled back as shown in FIG. 10B, causing the clamp jaws 112, 114 to open in preparation for surrounding the structure to be clamped (in this case, the aorta 226 which is schematically illustrated in cross-section in FIG. 10B.) While maintaining the pulling force on the opening suture ends 122, 124 as necessary to keep the jaws 112, 114 open, the introducer shaft 146 can be used to guide the clamp jaws 112, 114 around the structure to be clamped 226 as schematically illustrated in FIG. 10C.

As illustrated in FIG. 10D, the ends 136, 138 of the closing suture 134 are then pulled, causing the jaws 112, 114 to close and clamp down on the structure 226 to be clamped. The view of FIG. 10D shows the structure 226 partially clamped, and complete clamping may be achieved by further pulling of the closing suture 134 in conjunction with the application of a mechanical knot 228 to the locking suture 104. The mechanical knot 228 can be applied over the ends 106, 108 of the locking suture, for example with a Ti-KNOT® device or a COR-KNOT® device available from LSI Solutions, Inc. of Victor, NY. (For example, find ordering contact information at www.lsisolutions.com). Other embodiments of mechanical knots or other types of knots may be used with this or other embodiments of clamping devices. The mechanical knot applicator (known to those skilled in the art) pushes an uncrimped knot 228 over the ends 106, 108 of the lock down suture 104, cinching the knot 228 toward the clamp, thereby also helping to close the clamp jaws 112, 114 as the actuator arms 94, 96 are drawn together. The mechanical knot 228 can then be crimped to lock the locking suture 104 in place as shown in FIG. 10E. Since the locking suture 104 passes through first and second locking suture guides 100, 102, the clamp jaws are locked together by the locking suture 104 and mechanical knot 228 as shown in FIG. 10E. The mechanical knot 228 can provide a significant clamping force that ensures the clamp jaws 112, 114 do not come unclamped before the surgeon wants them to.

As illustrated in FIG. 10E, the introducer shaft can be removed after the clamp is locked in the clamped position. In this embodiment, this can be accomplished by detaching the tie-down suture 156 from the cleat (not shown) on the distal end of the introducer shaft 146. The shaft 146 can then be slid off of the introducer connection point 166 to which it was previously coupled. In this embodiment, the tie-down suture 156 also acts as a retrieval suture 156, and the end of the retrieval suture can be kept handy outside of the patient for later use in removing the clamp, after the clamp is released. In order to release the clamp, a surgeon can cut the lockdown suture 104, and then pull the clamp back directly or via the retrieval suture 156.

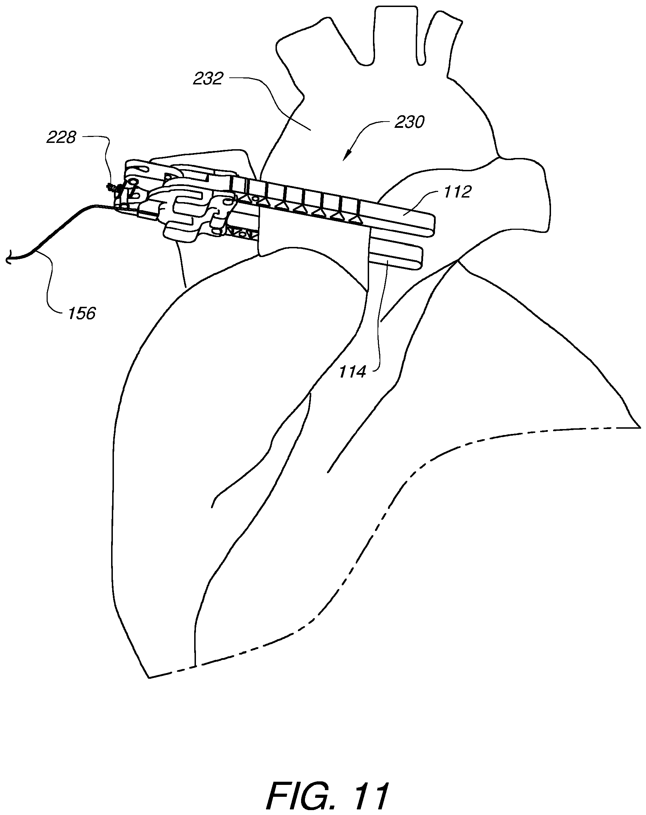

Embodiments of the surgical clamp device discussed herein, and their equivalents, may be advantageously used as an aortic cross clamp. FIG. 11 illustrates one such embodiment 230 locked in a clamped position across a section of the aorta 232 by a mechanical knot 228. Since the introducer shaft can be removed, leaving only a retrieval suture 156 to pull the clamp out (once released), the impact to a limited access surgical access site is minimal, enabling surgeons to have more access for tools and other implements during a related surgical procedure. Furthermore, the introducer shaft does not need to be reattached in order to remove the clamp when finished.

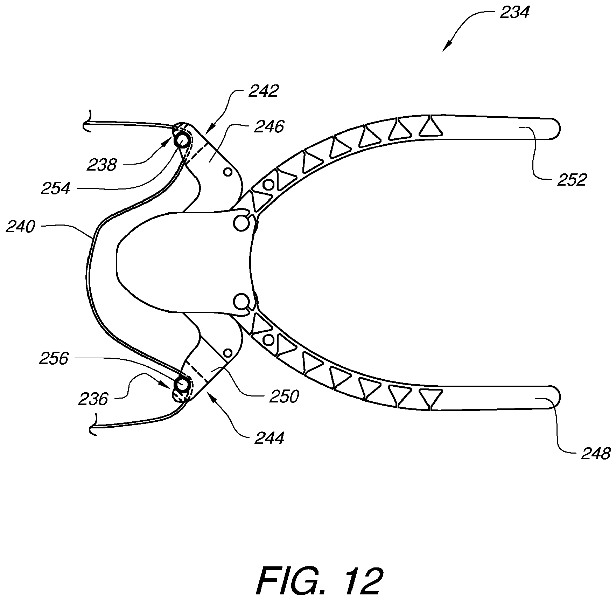

As mentioned previously, removal of the surgical clamp device may be accomplished by first cutting the locking suture which is held by the mechanical knot. When doing this, however, there may be a chance in some embodiments that the knot and locking suture that has been cut will fall out of the locking suture guides. The surgeon will need to make sure that the locking suture and knot are retrieved as well as the clamp. Therefore, as discussed in the embodiments of FIGS. 12-14, it may also be desirable to provide at least one suture retainer to the surgical clamping device. For example, FIG. 12 is a top view of a surgical clamping device 234 having one embodiment of suture retainers 236, 238. A locking suture 240 is shown threaded through a first locking suture guide 242 and then through a second locking suture guide 244 as previously discussed. In this embodiment, the first locking suture guide 242 is a channel formed in the actuator arm 246 on the end of clamp jaw 248. Likewise, the second locking suture guide 244 is a channel formed in the actuator arm 250 on the end of clamp jaw 252. The actuators 246, 250 in this embodiment also include a pin 254, 256, respectively, to help provide a smooth wrap-around surface for the lockdown suture 240. As the lockdown suture 240 is tightened and locked into place, the lockdown suture 240 will be drawn into the suture retainers 236, 238. The suture retainers 236, 238 are configured to resist separation of the lockdown suture 240 from the first or second clamp jaws 248, 252. In this embodiment, the suture retainers comprise a tapered channel or groove which the suture can be pressed into during the application of the mechanical knot.

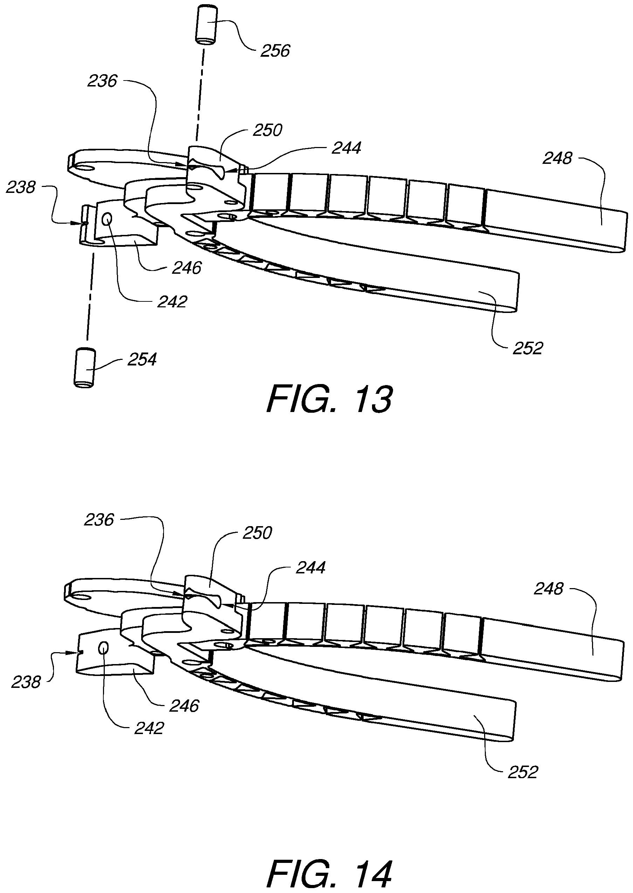

The channel type structure of retainers 236, 238 can be seen more clearly in the partially exploded perspective view of FIG. 13. For clarity, the lower portion of the housing is not shown in FIG. 13, and the pins 254, 256 have been exploded apart from the jaw actuator arms 246, 250. FIG. 14 illustrates a similar embodiment having suture retainers 236, 238 but without the pins 254, 256. In both embodiments, the suture retainers 236, 238 are sized to pinch the locking suture (not shown in these views) when the mechanical knot is applied to lock the locking suture in place to keep the clamp jaws 248, 252 in a clamped position.

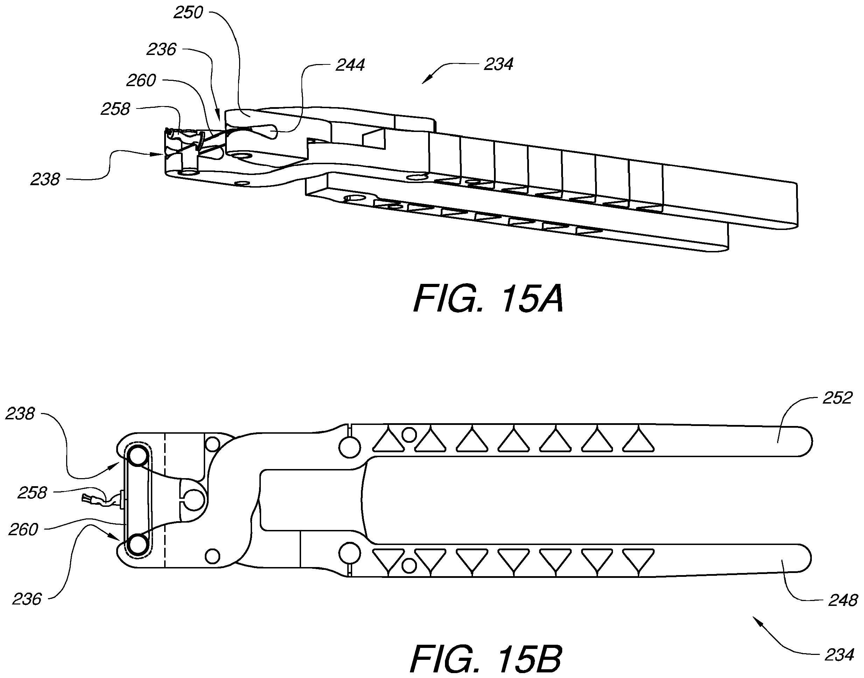

FIGS. 15A and 15B are perspective and top views, respectively, of the surgical clamping device 234 from FIGS. 12-13 having suture retainers 236, 238 and locked in a clamped position by a mechanical knot 258. Portions of the housing are removed from each view for clarity. The locking suture 260 can be seen pinched in the suture retainers 236, 238. Although this embodiment includes two suture retainers, other embodiments may have a greater or lesser number of suture retainers.

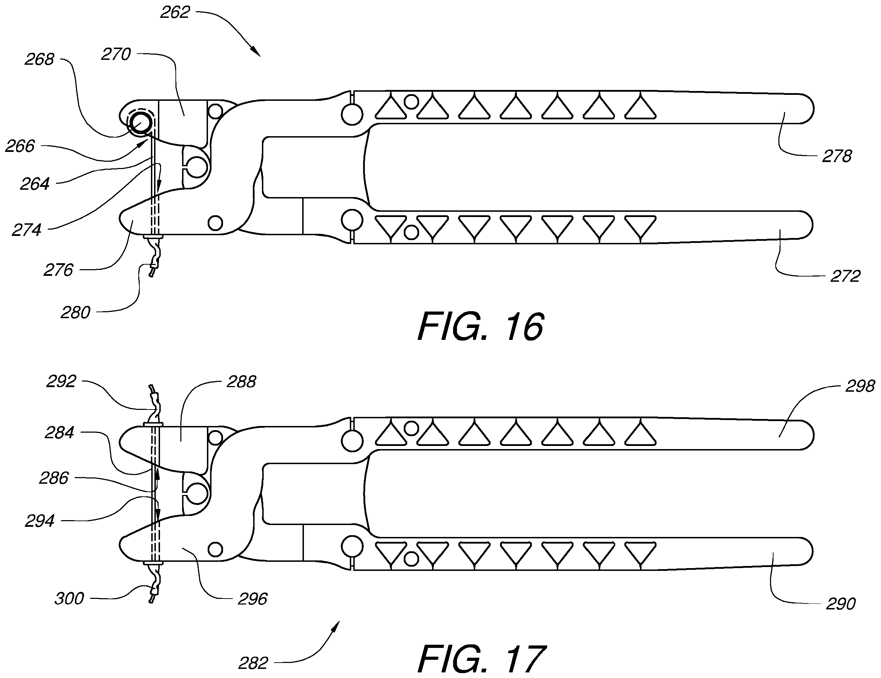

All of the surgical clamp embodiments discussed to this point have first and second locking suture guides configured to receive at least one locking suture for knotting to hold the first and second clamp jaws in a clamped position. Until this point, the locking suture has been in a continuous loop held together by a mechanical knot. FIGS. 16 and 17 illustrate some other possible non-limiting embodiments. FIG. 16 shows a clamping device 262 having a locking suture 264 passed through a first locking suture guide 266 and tied or looped around a pin 268 on a first actuator arm 270 coupled to clamp jaw 272. The locking suture 264 passes through a second locking suture guide 274 in a second actuator arm 276 coupled to clamp jaw 278. The clamp jaws 272, 278 are shown in a clamped position, although, for clarity, a structure being clamped between the jaws and portions of the housing are not shown. The jaws 272, 278 are locked in this clamped position by a mechanical knot 280 fastened to a single strand of locking suture 264.

The surgical clamping device 282 of FIG. 17 has a locking suture 284 passed through a first locking suture guide 286 in a first actuator arm 288 coupled to a clamp jaw 290. The locking suture 284 is anchored in place by a first mechanical knot 292 where the locking suture 284 exits the first locking suture guide 286. Likewise, the locking suture 284 is also passed through a second locking suture guide 294 in a second actuator arm 296 coupled to a second clamp jaw 298. The clamp jaws 290, 298 are shown in a clamped position, although, for clarity, a structure being clamped between the jaws and portions of the housing are not shown. The jaws 290, 298 are locked in this clamped position by a second mechanical knot 300 (working in conjunction with the first mechanical knot 292) fastened to the locking suture 284 where it exits the second locking suture guide 294.

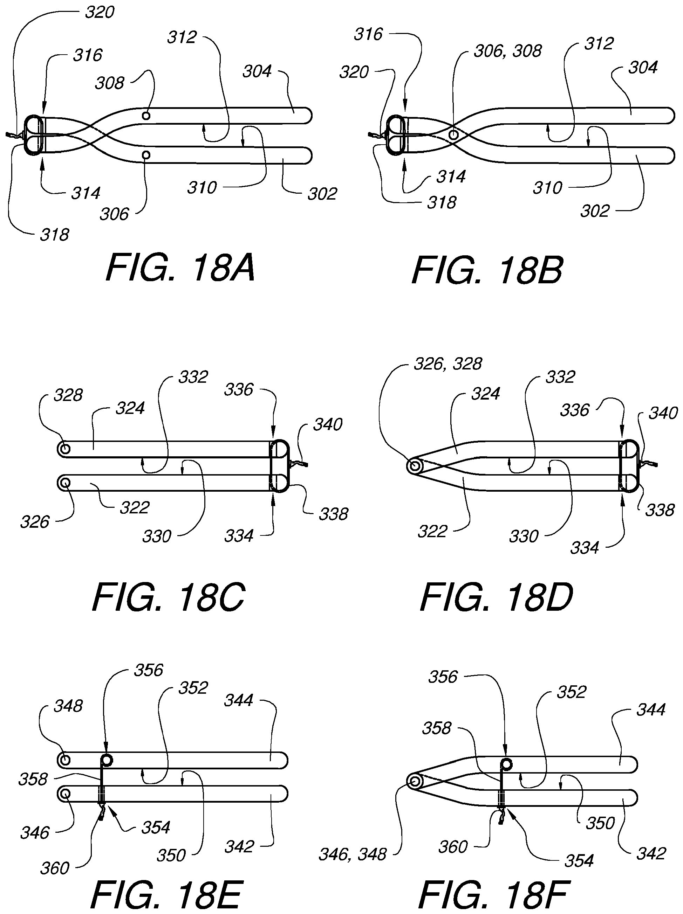

A variety of clamp jaw configurations are possible with the surgical device embodiments described herein. For ease of explanation, the embodiments to this point have had a similar relationship between the pivot points, the clamping surfaces, and the locking suture guides. For example, the embodiments to this point have had an arrangement similar to the clamp schematically illustrated in FIG. 18A. In FIG. 18A, the first and second clamp jaws 302, 304 have separate pivot points 306, 308 which do not share a common pivot axis. The first and second clamp jaws 302, 304 also have first and second clamping surfaces 310, 312, respectively. The first and second clamp jaws 302, 304 further have first and second locking suture guides 314, 316, respectively. A locking suture 318 is threaded through the first and second locking suture guides 314, 316 and a mechanical knot 320 is holding the first and second clamp jaws 302, 304 in a clamped position. For clarity, a housing and a structure being clamped are not shown in this view. In this embodiment, the pivot points 306, 308 are located between their respective clamping surfaces 310, 312, and their respective locking suture guides 314, 316. The embodiment of FIG. 18B is similar to the embodiment of FIG. 18A, except that the first and second clamp jaws 302, 304 have respective pivot points 306, 308 that share a common pivot axis.

In FIG. 18C, the first and second clamp jaws 322, 324 have separate pivot points 326, 328 which do not share a common pivot axis. The first and second clamp jaws 322, 324 also have first and second clamping surfaces 330, 332, respectively. The first and second clamp jaws 322, 324 further have first and second locking suture guides 334, 336, respectively. A locking suture 338 is threaded through the first and second locking suture guides 334, 336 and a mechanical knot 340 is holding the first and second clamp jaws 322, 324 in a clamped position. For clarity, a housing and a structure being clamped are not shown in this view. In this embodiment, the clamping surfaces 330, 332 are located between their respective pivot points 326, 328, and their respective locking suture guides 334, 336. The embodiment of FIG. 18D is similar to the embodiment of FIG. 18C, except that the first and second clamp jaws 322, 324 have respective pivot points 326, 328 that share a common pivot axis.

In FIG. 18E, the first and second clamp jaws 342, 344 have separate pivot points 346, 348 which do not share a common pivot axis. The first and second clamp jaws 342, 344 also have first and second clamping surfaces 350, 352, respectively. The first and second clamp jaws 342, 344 further have first and second locking suture guides 354, 356, respectively. A locking suture 358 is threaded, looped, or tied around the second locking suture guide 356 and then threaded through the first locking suture guide 354. A mechanical knot 360 is holding the first and second clamp jaws 342, 344 in a clamped position. For clarity, a housing and a structure being clamped are not shown in this view. In this embodiment, the locking suture guides 354, 356 are located between their respective pivot points 346, 348, and their respective clamping surfaces 350, 352. The embodiment of FIG. 18F is similar to the embodiment of FIG. 18E, except that the first and second clamp jaws 342, 344 have respective pivot points 346, 348 that share a common pivot axis.

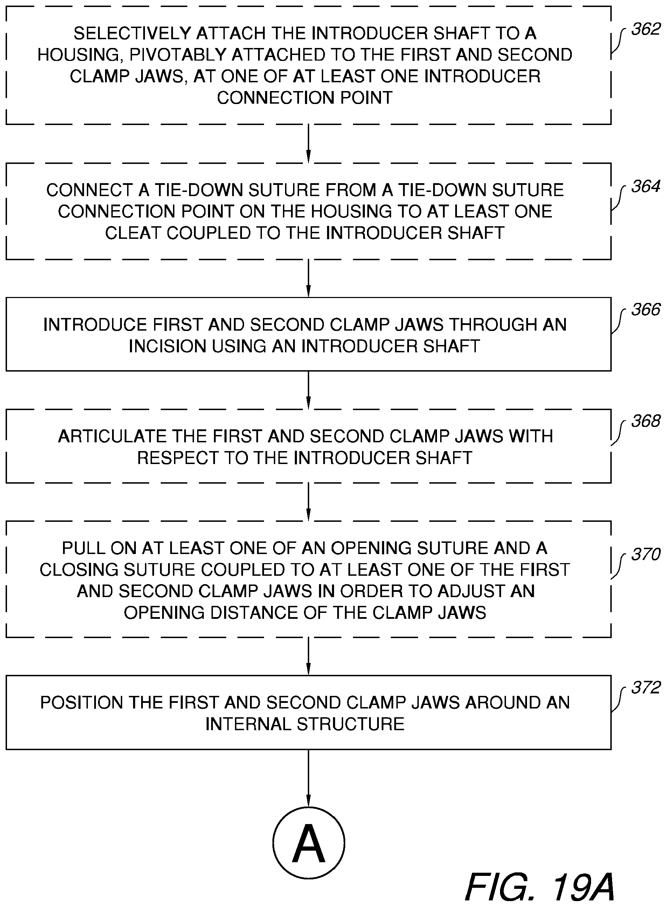

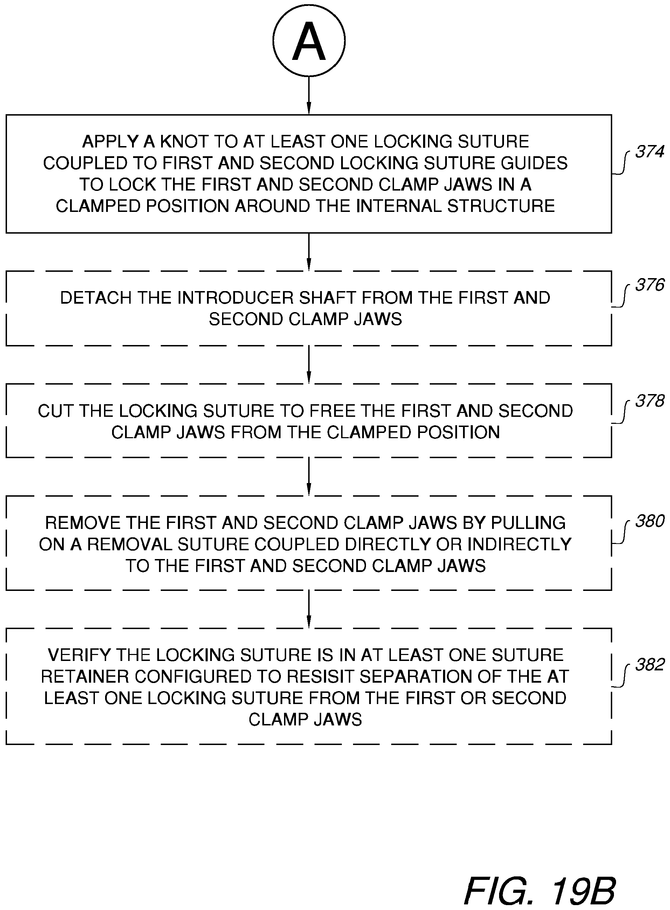

FIGS. 19A-19B illustrate one embodiment of a method for clamping a structure during surgery. In optional step 362, an introducer shaft may be selectively attached to a housing, pivotably attached to first and second clamp jaws, at one of at least one introducer connection point. In optional step 364, a tie-down suture is connected from a tie-down suture connection point on the housing to at least one cleat coupled to the introducer shaft. A cleat may be any structure intended to retain the tie-down suture. Steps 362 and 364 are optional because some embodiments may not have a removable introducer shaft and/or a tie-down suture.

In step 366, first and second clamp jaws are introduced through an incision using an introducer shaft. In optional step 368, the first and second clamp jaws are articulated with respect to the introducer shaft. In optional step 370, at least one of an opening suture and a closing suture coupled to at least one of the first and second clamp jaws are pulled on in order to adjust an opening distance of the clamp jaws.

In step 372, the first and second clamp jaws are positioned around an internal structure. In step 374, a knot is applied to at least one locking suture coupled to first and second locking suture guides to lock the first and second clamp jaws in a clamped position around the internal structure. In some embodiments, the knot may be a mechanical knot.

In optional step 376, the introducer shaft may be detached from the first and second clamp jaws, for example, by detaching from a housing to which the jaws are pivotably attached. Various structures for doing this have been discussed above, including, but not limited to detaching a tie-down suture. In a further optional step 378, if it is desired to release the clamp, the locking suture may be cut to free the first and second clamp jaws from the clamped position. In optional step 380, the first and second clamp jaws may be removed by pulling on a removal suture coupled directly or indirectly to the first and second clamp jaws. The removal suture could be a tie-down suture, an opening suture, a closing suture, or a separate suture for that purpose, depending on the embodiment.

In optional step 382, the cut locking suture is verified to be in at least one suture retainer configured to resist separation of the at least one locking suture from the first or second clamp jaws. This action may be helpful to verify that no portion of the clamping device has been left behind in the patient.

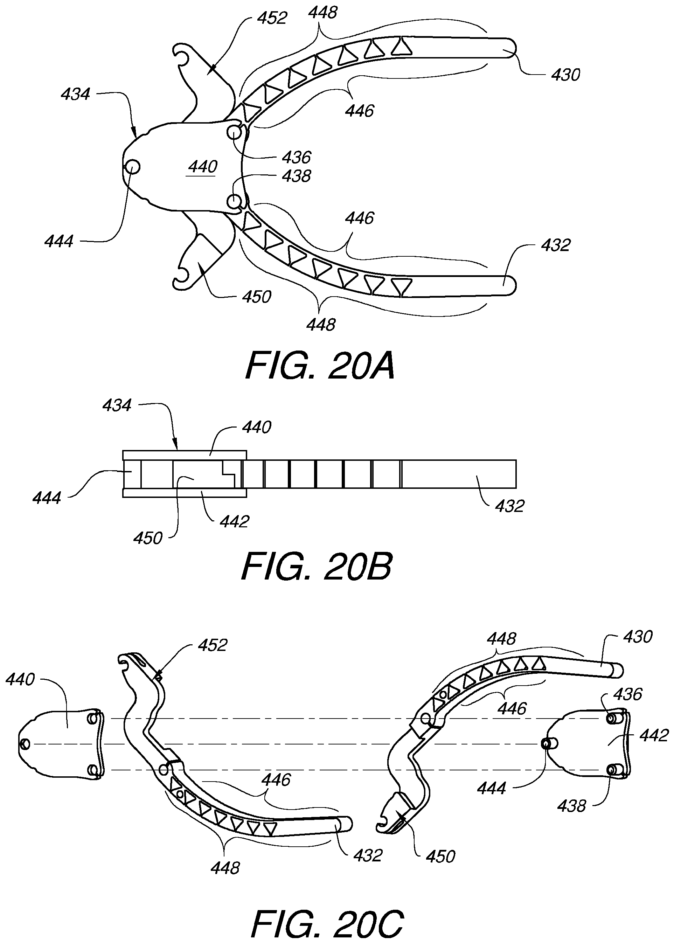

FIG. 20A is a top view of one embodiment of surgical clamp jaws 430, 432 pivotably held by a housing 434. The clamp jaw 430 pivots around pivot point 436, while clamp jaw 432 pivots around pivot point 438. FIGS. 20B and 20C show the assembly of FIG. 20A in side and exploded views, respectively, in order to better illustrate the embodiment. The housing 434 in this embodiment has a top plate 440 and a bottom plate 442. In addition to locating the pivot points 436 and 438, the housing plates 440, 442 may also be coupled by one or more supports 444.

Each surgical clamp jaw 430, 432 has an inner profile 446 and a deflection control profile 448 opposite the inner profile 446. The deflection control profile 448 may be configured to allow the inner profile 446 to have one shape when the clamp jaws 430, 432 are in an unclamped position and another shape when the clamp jaws 430, 432 are in a clamped position. Various embodiments of the inner profile 446 and the deflection control profile 448 will be discussed later in this specification.

Since the surgical clamp jaws 430, 432 each are pivotable around their respective pivot points 436, 438, each jaw 430, 432 may be coupled to an actuator 450, 452 configured to rotate the respective inner profile 446 of each surgical clamp jaw 430, 432 around its respective pivot point 436, 438. Some examples of actuators 450, 452 may include, but are not limited to levers, arms, gears, pulleys, motors, or any combination or plurality thereof. Such actuators are well known to those skilled in the art and therefore, the actuators illustrated and discussed herein are often shown as simple arms, such as arms 450, 452, or the like, for simplicity. It should be understood, however, that a wide variety of actuators and their equivalents are intended to be covered herein.

In the orientation of FIG. 20A, the actuator 450 can be rotated in a clockwise arc around pivot point 436 to move surgical clamp jaw 430 in a similar direction towards surgical clamp jaw 432. Likewise, the actuator 452 can be rotated in a counterclockwise arc around pivot point 438 to move surgical clamp jaw 432 in a similar direction towards surgical clamp jaw 430. The clamp jaws 430, 432 can also be moved apart from each other by reversing the direction of the actuators.

The deflection control profile 448, opposite the inner profile 446, is an important concept for the embodiments disclosed herein. FIG. 21A illustrates one embodiment of a surgical clamp jaw 516 in an unclamped position. The surgical clamp jaw 516 has an inner profile 518 and a deflection control profile 520 opposite the inner profile 518. Although a portion 522 of the clamp jaw has a straight profile in this embodiment, when taking into account the totality of the inner profile 518, the inner profile 518 still has a substantially concave profile in the unclamped position. The clamp jaw 516 also has a pivot point 524 and an arm 526 which can be used as an actuator or coupled to another actuator.

In this embodiment, the deflection control profile 520 comprises one or more sets of corresponding abutment surfaces which are best seen in the enlarged view of FIG. 21B. FIG. 21B shows a first set of corresponding abutment surfaces 528A, 528B and a second set of corresponding abutment surfaces 530A, 530B. For convenience, only one set of corresponding abutment surfaces 528A, 528B will be discussed, however, it should be understood that the other sets of corresponding abutment surfaces will operate in a similar fashion. In the unclamped position shown in FIG. 21B, the set of corresponding abutment surfaces 528A, 528B are not contacting each other. Instead, they are separated by an abutment separation distance 532. Depending on the embodiment, the abutment separation distance 532 between each set of corresponding abutment surfaces 528A, 528B may be the same or different. As the surgical clamp jaw 516 is moved from an unclamped position (shown in FIG. 21A) to a clamped position (shown in FIG. 21C), the inner profile 518 will be able to deflect back towards the deflection control profile 520 until the abutment surfaces 528A, 528B come into contact with each other. A clamping force 534, from the clamp jaw 516 acting in concert with another clamp jaw (not shown, but discussed previously), acts on the clamp jaw 516 in order cause the deflection. The abutment separation distance 532 can be established to control the amount of deflection possible for the inner profile 516. Smaller abutment separation 532 will enable less deflection, while larger abutment separation 532 will enable more deflection. In this embodiment, the inner profile 518 is substantially flat in the clamped position, as illustrated in FIG. 21C.

In order for the inner profile 518 to be able to deflect until the corresponding abutment surfaces 528A, 528B contact each other, some embodiments may include one or more flexion assistance voids 536. The flexion assistance voids 536 reduce the effective thickness 538 of the clamp jaw 516 in certain places behind the inner profile 518, thereby making the inner profile 518 more flexible. In the embodiment of FIGS. 21A-21C, the flexion assistance voids 536 have a substantially triangular shape, although other embodiments may use other shapes. Also, in this embodiment, each flexion assistance void 536 is in contact with the gap 532 between the set of corresponding abutment surfaces 528A, 528B. This continuity between the gap 532 and the flexion assistance void 536 may be desirable from a manufacturing point of view, but it is not necessary in all embodiments.

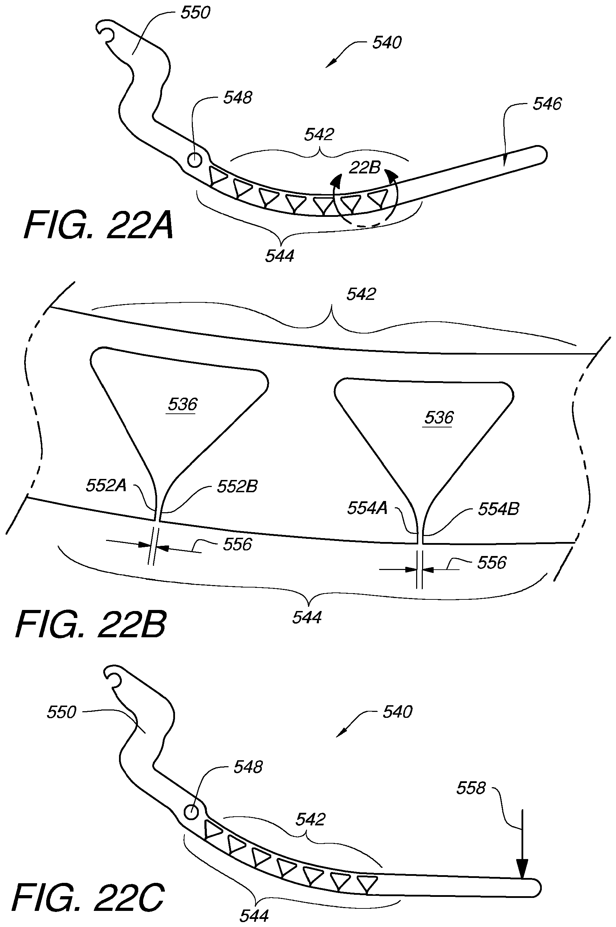

FIG. 22A illustrates another embodiment of a surgical clamp jaw 540 in an unclamped position. The surgical clamp jaw 540 has an inner profile 542 and a deflection control profile 544 opposite the inner profile 542. Although a portion 546 of the clamp jaw 540 has a straight profile, in this embodiment, when taking into account the totality of the inner profile 542, the inner profile 542 has a first substantially concave profile in the unclamped position. The clamp jaw 540 also has a pivot point 548 and an arm 550 which can be used as an actuator or coupled to another actuator.

As with the previous embodiment, in this embodiment, the deflection control profile 544 comprises one or more sets of corresponding abutment surfaces which are best seen in the enlarged view of FIG. 22B. FIG. 22B shows a first set of corresponding abutment surfaces 552A, 552B and a second set of corresponding abutment surfaces 554A, 554B. For convenience, only one set of corresponding abutment surfaces 552A, 552B will be discussed, however, it should be understood that the other sets of corresponding abutment surfaces will operate in a similar fashion. In the unclamped position shown in FIG. 22B, the set of corresponding abutment surfaces 552A, 552B are not contacting each other. Instead, they are separated by an abutment separation distance 556. The abutment separation distance 556 in the embodiment of FIGS. 22A-22C is smaller than the abutment separation distance 532 from the embodiment of FIGS. 21A-21C. As a result, by comparison, the embodiment illustrated in FIGS. 22A-22C is not able to deflect as far. Accordingly, as the surgical clamp jaw 540 is moved from an unclamped position (shown in FIG. 22A) to a clamped position (shown in FIG. 22C), the inner profile 542 will be able to deflect back towards the deflection control profile 544 until the abutment surfaces 552A, 552B come into contact with each other, resulting in the inner profile 542 having a second substantially concave profile in the clamped position of FIG. 22C. While having a concave profile in the clamped position may not be useful for completely occluding some conduits, the concave clamped profile may allow a surgeon to partially occlude a conduit. Such a clamp could be used in conjunction with a completely occluding clamp in order to help avoid sudden pressure changes inside the conduit. For example, the conduit could be partially occluded with one clamp and then completely occluded with a second clamp, each clamp having differing inner profiles in the clamped position. Near the end of the surgical procedure, the completely occluding clamp could be removed first, allowing some fluid to flow through the partially occluded clamp. This might allow the surgeon to ease the patient's related biological systems into full use as the partially occluded clamp would later be released.

As with the previous embodiment, a clamping force 558, from the clamp jaw 540 acting in concert with another clamp jaw (not shown, but discussed previously) acts on the clamp jaw 540 in order cause the deflection. The clamp jaw 540 in this embodiment also has flexion assistance voids 536, the features of which have been discussed previously.

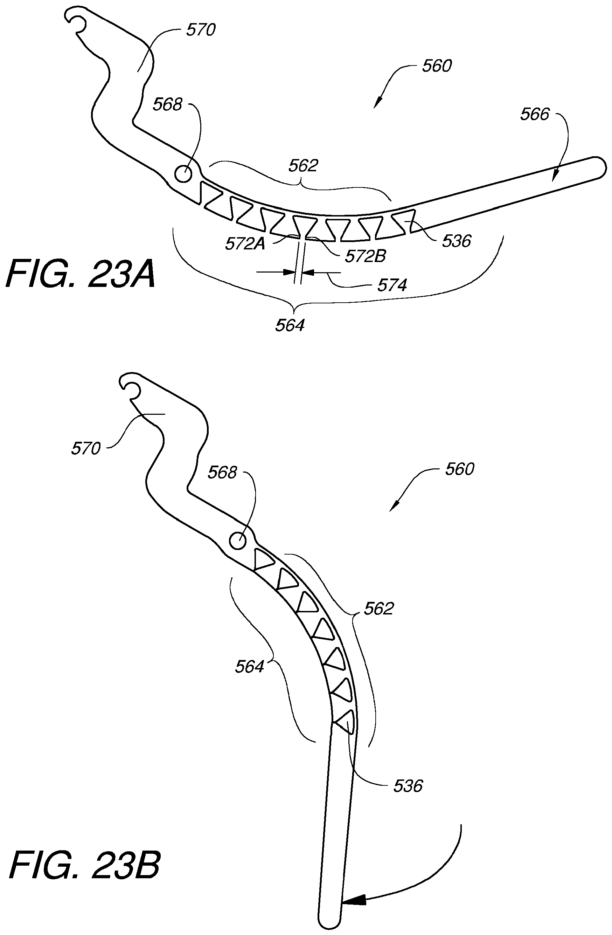

FIG. 23A illustrates another embodiment of a surgical clamp jaw 560 in an unclamped position. The surgical clamp jaw 560 has an inner profile 562 and a deflection control profile 564 opposite the inner profile 562. Although a portion 566 of the clamp jaw 540 has a straight profile in this embodiment, when taking into account the totality of the inner profile 562, the inner profile 562 has a substantially concave profile in the unclamped position. The clamp jaw 560 also has a pivot point 568 and an arm 570 which can be used as an actuator or coupled to another actuator.

As with the previous embodiments, in this embodiment, the deflection control profile 564 comprises one or more sets of corresponding abutment surfaces. For convenience, only one set of corresponding abutment surfaces 572A, 572B will be discussed, however it should be understood that the other sets of corresponding abutment surfaces will operate in a similar fashion. In the unclamped position shown in FIG. 23A, the set of corresponding abutment surfaces 572A, 572B are not contacting each other. Instead, they are separated by an abutment separation distance 574. The abutment separation distance 574 in the embodiment of FIGS. 23A-23B is larger than the abutment separation distance 532 from the embodiment of FIGS. 21A-21C. As a result, by comparison, the embodiment of FIGS. 23A-23B is able to deflect farther. Accordingly, as the surgical clamp jaw 560 is moved from an unclamped position (shown in FIG. 23A) to a clamped position (shown in FIG. 23B), the inner profile 562 will be able to deflect back towards the deflection control profile 564 until the abutment surfaces 572A, 572B come into contact with each other, resulting in the inner profile 562 having a substantially convex profile in the clamped position of FIG. 23B. While having a convex inner profile in the clamped position would not be useful in many situations, such a clamp might be useful where softer gripping forces are needed or where the clamp had to be used to hold or steady an unusually shaped structure. The clamp jaw 560 in this embodiment also has flexion assistance voids 536, the features of which have been discussed previously.

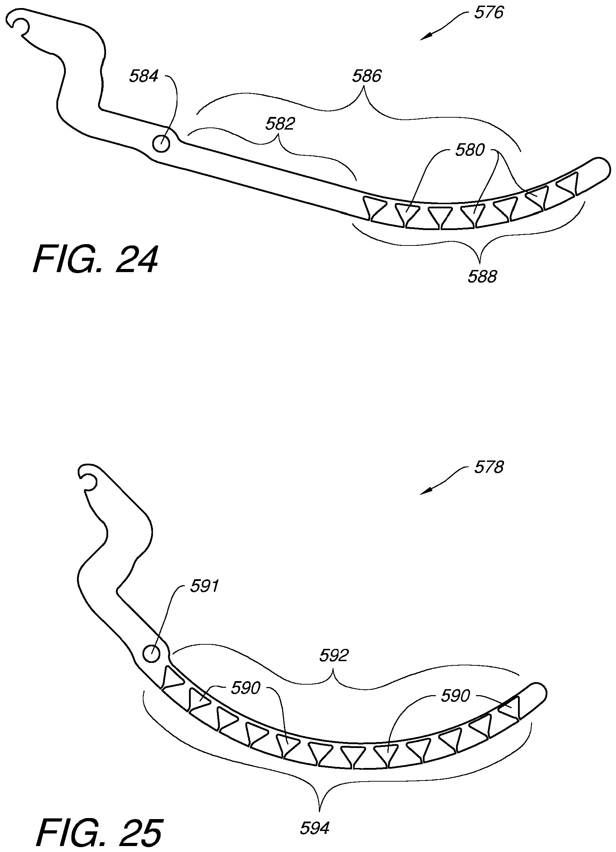

In the embodiments discussed up to this point, the flexion assistance voids 536 have been located in a section of the surgical clamp jaws starting near the pivot point and ending before a straight section at the tip of the clamp. Other embodiments may have different distributions of flexion assistance voids. As just two examples, FIGS. 24 and 25 illustrate embodiments of surgical clamp jaws 576 and 578 having examples of different flexion assistance void distribution. In FIG. 24, surgical clamp jaw 576 has flexion assistance voids 580 which are located near to the tip of the clamp jaw 576, while the clamp jaw 576 also has a straight section 582 nearer to the pivot point 584. Even with the straight section 582, the clamp jaw 576 still has a substantially concave inner profile 586 in the unclamped position illustrated in FIG. 24. The clamp jaw 576 also has a deflection control profile 588 opposite the inner profile 586. The features of deflection control profiles have been discussed previously.

In FIG. 25, surgical clamp jaw 578 has flexion assistance voids 590 which are distributed continuously between the pivot point 591 and the tip of the clamp jaw 578. The clamp jaw 578 has a substantially concave inner profile 592 in the unclamped position illustrated in FIG. 25. The clamp jaw 578 also has a deflection control profile 594 opposite the inner profile 592. The features of deflection control profiles have been discussed previously.

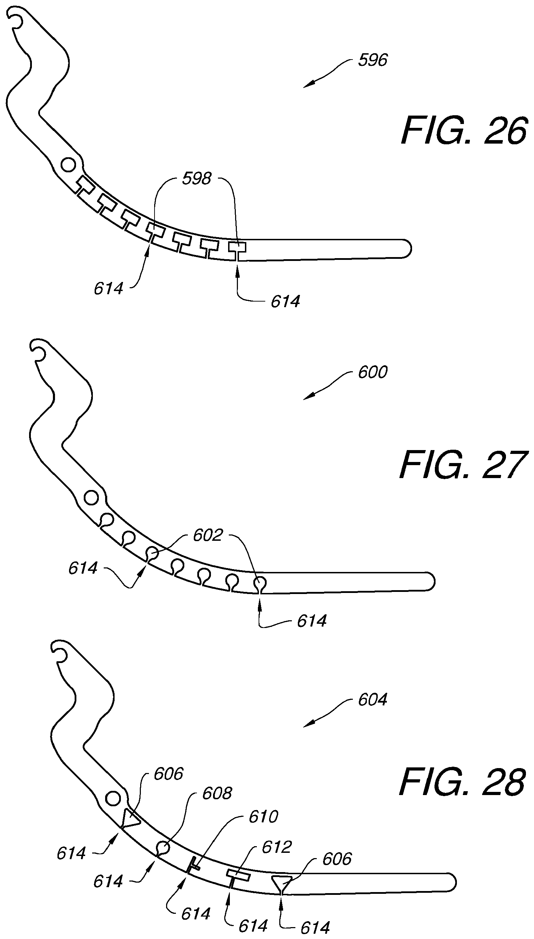

FIGS. 26-28 illustrate further embodiments of surgical clamp jaws featuring examples of different flexion assistance void shapes. Up to this point, the flexion assistance voids have been illustrated as substantially triangular, however, as has been noted above, the flexion assistance voids are not limited to one particular shape. For example, as with the surgical clamp jaw 596 illustrated in FIG. 26, the flexion assistance voids 598 are substantially rectangular. As another example, the surgical clamp jaw 600 illustrated in FIG. 27 has flexion assistance voids 602 which are substantially circular. Depending on the embodiment, the shapes of flexion assistance voids in a given surgical clamp jaw do not have to be uniform. As just one example, the surgical clamp jaw 604 illustrated in FIG. 28 has substantially triangular flexion assistance voids 606, a substantially circular flexion assistance void 608, and differently sized substantially rectangular assistance voids 610, 612. Other flexion assistance void shapes may be used in other embodiments.

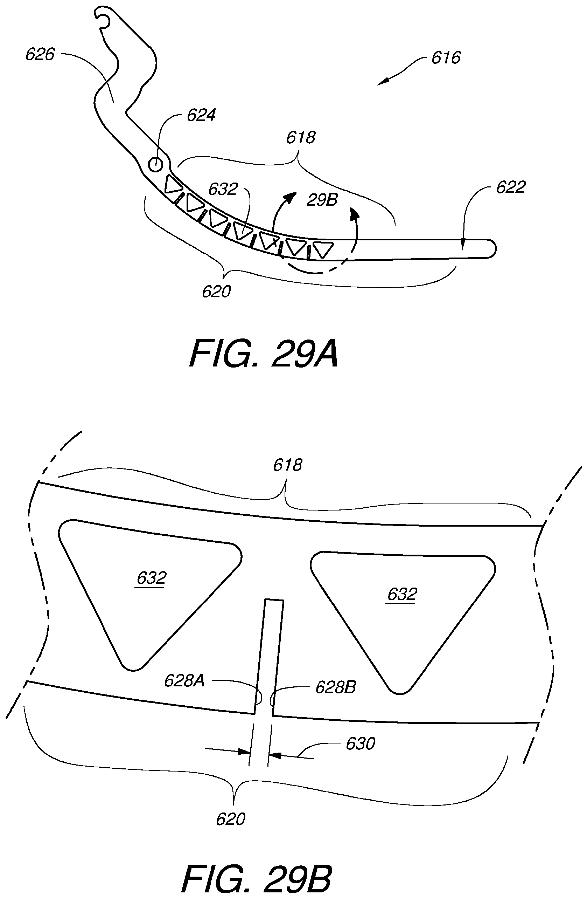

In the embodiments of FIGS. 26-28, each flexion assistance void is in contact with a gap 614 between a set of corresponding abutment surfaces. However, depending on the embodiment, a flexion assistance void does not need to be in contact with a gap between corresponding abutment surfaces. For example, FIG. 29A illustrates one embodiment of a surgical clamp jaw 616 in an unclamped position. The surgical clamp jaw 616 has an inner profile 618 and a deflection control profile 620 opposite the inner profile 616. Although a portion 622 of the clamp jaw 616 has a straight profile, in this embodiment, when taking into account the totality of the inner profile 618, the inner profile 618 has a substantially concave profile in the unclamped position. The clamp jaw 616 also has a pivot point 624 and an arm 626 which can be used as an actuator or coupled to another actuator.

In this embodiment, the deflection control profile 620 comprises one or more sets of corresponding abutment surfaces which are best seen in the enlarged view of FIG. 29B. FIG. 29B shows a set of corresponding abutment surfaces 628A, 628B. For convenience, only one set of corresponding abutment surfaces 628A, 628B will be discussed, however it should be understood that the other sets of corresponding abutment surfaces will operate in a similar fashion. In the unclamped position shown in FIG. 29B, the set of corresponding abutment surfaces 628A, 628B are not contacting each other. Instead, they are separated by an abutment separation distance 630. Depending on the embodiment, the abutment separation distance 630 between each set of corresponding abutment surfaces 628A, 628B may be the same or different. As the surgical clamp jaw 616 is moved from an unclamped position (shown in FIG. 29A) to a clamped position (not shown), the inner profile 618 will be able to deflect back towards the deflection control profile 620 until the abutment surfaces 628A, 628B come into contact with each other. As with previous embodiments, the abutment separation distance 630 can be established to control the amount of deflection possible for the inner profile 618. In this embodiment, the surgical clamp jaw 616 also has flexion assistance voids 632 which are not in contact with the gap 630 between a set of corresponding abutment surfaces 628A, 628B. The flexion assistance voids 632 will still serve to increase the flexibility of the inner profile 618.

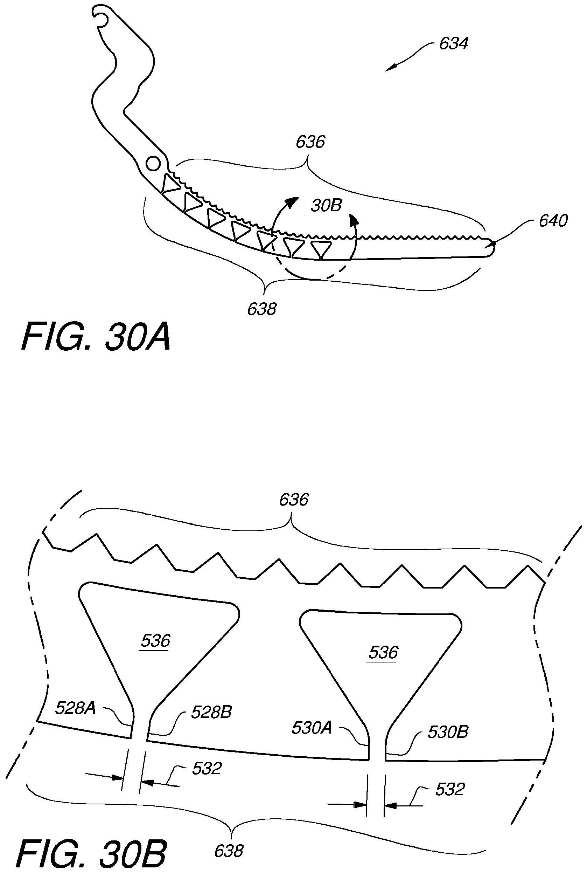

Although the inner profiles of the surgical clamp jaws illustrated to this point have had a smooth surface, other embodiments may have a rough surface for the inner profile. For example, FIG. 30A illustrates one embodiment of a surgical clamp jaw 634 in an unclamped position. The surgical clamp jaw 634 has an inner profile 636 and a deflection control profile 638 opposite the inner profile 636. In this embodiment, the inner profile 636 is textured. This could be useful, for example, to increase the grip of the inner profile 636.

Although a portion 640 of the clamp jaw is straight in this embodiment, when taking into account the totality of the inner profile 636, the inner profile 636 still has a substantially concave profile in the unclamped position. FIG. 30B shows an enlarged view of a portion of the surgical clamp jaw 634 of FIG. 30A. The remainder of the features of the surgical clamp jaw 634 are similar to the embodiments discussed previously and have corresponding element numbers.

The advantages of having a surgical clamp jaw with a concave inner profile in the unclamped position have been discussed above. These advantages include, but are not limited to, helping to prevent a conduit from being pushed out of the clamp as the clamp is tightened into a clamped position and helping to prevent the conduit from popping out of the clamp too soon as the clamp is opened (thereby giving surgeons more control over the release of the clamp).

According to an embodiment of the invention, however, it is desirable to replace or supplement the actuator locking features with interlocking features located in one or more sets of corresponding abutment surfaces of the deflection control profile. FIG. 31A illustrates an embodiment of the invention of a surgical clamp jaw 642 having interlocking features (discussed below) on corresponding abutment surfaces. The surgical clamp jaw 642 has an inner profile 644 and a deflection control profile 646 opposite the inner profile 644. The inner profile 644 has a substantially concave profile in the unclamped position. The clamp jaw 642 also has a pivot point 648 and an arm 650 which can be used as an actuator or coupled to another actuator.

In this embodiment, the deflection control profile 646 comprises one or more sets of corresponding abutment surfaces which are best seen in the alternate enlarged views of FIGS. 31B-1 and 31B-2. The features of corresponding abutment surfaces have been discussed previously. Therefore, for convenience, only one set of corresponding abutment surfaces 652A, 652B will be discussed. It should be understood, however, that the other sets of corresponding abutment surfaces will operate in a similar fashion. In the embodiments illustrated in FIGS. 31B-1 and 31B-2, the first abutment surface 652A has a first interlocking feature 654A, while the second abutment surface 652B has a second interlocking feature 654B. In the embodiment of FIG. 31B-1, and according to the invention, the first and second interlocking features 654A, 654B are not in contact with each other when the inner profile 644 is in an unclamped position. In the example of FIG. 31B-2, not forming part of the invention, the first and second interlocking features 654A, 654B are contacting each other when the inner profile 644 is in an unclamped position. In either case, in the unclamped position, the abutment surfaces 652A, 652B are still separated and the first and second interlocking features are not interlocked.

As the surgical clamp jaw 642 is moved from an unclamped position (shown in FIG. 31A) to a clamped position (not shown), the inner profile 644 will be able to deflect back towards the deflection control profile 646 until the abutment surfaces 652A, 652B come into contact with each other. As the corresponding abutment surfaces 652A, 652B come together, the corresponding interlocking features 654A, 654B will also be forced together into an interlocking arrangement. This can help to offset the tendency of the inner profile 644 to want to return to a concave position, which may be desirable in some situations.

FIG. 32A illustrates a further embodiment according to the invention of a surgical clamp jaw 656 having a different arrangement of interlocking features 658A, 658B on corresponding abutment surfaces 660A, 660B. These features are best seen in the enlarged view of FIG. 32B which highlights a portion of the surgical clamp jaw 656 of FIG. 32A. The interlocking features 658A, 658B in this embodiment are oriented approximately ninety degrees from the interlocking features 654A, 654B of the previous embodiment. After seeing these examples, those skilled in the art will appreciate that other types of interlocking features in corresponding abutment surfaces are possible.

Up to this point, the surgical clamp jaw embodiments have been discussed and shown as if the inner profile of the clamp jaw would be in direct contact with any tissue that it is clamping. While such embodiments are very useful, it may also be advantageous to provide a shod (in this case a covering) for at least a portion of the clamp jaw. As one example, FIG. 33A illustrates the embodied surgical clamp jaw 516 of FIG. 21A (previously discussed), in an unclamped position, with one embodiment of a shod 662. The shod 662 has an opening 664 on a first end where the clamp jaw 516 may be inserted. In this embodiment, the opposite end 665 of the shod 662 is closed. FIG. 33B illustrates the embodied surgical clamp jaw 516 of FIG. 33A in a clamped position. The shod 662 is preferably flexible enough to move with the inner profile 518 as it changes shape moving from the unclamped position to the clamped position. The shod 662 (and all shod embodiments to be discussed herein) may be made from a wide variety of materials, including, but not limited to plastics, rubber, silicone, polymers, thermoplastics, resins, fabric, cotton, and fibers.

FIG. 34A illustrates the embodied surgical clamp jaw 516 of FIG. 21A (previously discussed), in an unclamped position, with another embodiment of a shod 668. The shod 668 has a first opening 670 on a first end where the clamp jaw 516 may be inserted. In this embodiment, the shod 668 also has a second opening 672 in a second end. In some embodiments, the second opening 672 may be a by-product of the fact that the shod could be manufactured from tubing that is cut to a particular length. In other embodiments, the second opening 672 may be specifically molded or formed. The second opening 672 can have the advantage of making the shod 668 easer to put on the surgical clamp jaw 516 since air cannot be caught and/or compressed into a closed end of the shod 668. FIG. 34B illustrates the embodied surgical clamp jaw 516 of FIG. 34A in a clamped position. The shod 668 is preferably flexible enough to move with the inner profile 518 as it changes shape moving from the unclamped position to the clamped position.

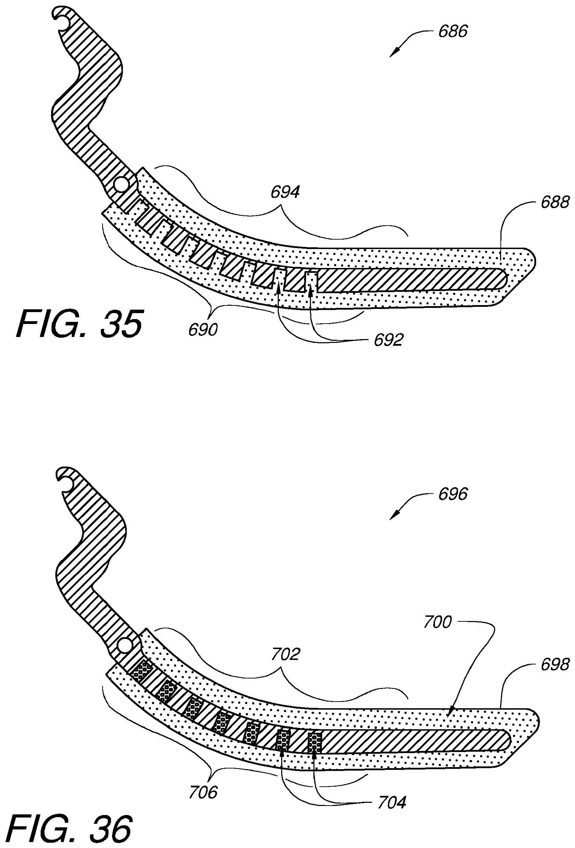

At the beginning of this specification, one embodiment of a surgical clamp jaw was described as having an inner profile and a deflection control profile opposite the inner profile. In the ensuing embodiments discussed up to this point, the deflection control profile included one or more sets of corresponding abutment surfaces which are not in contact with each other when the inner profile is in an unclamped position, but are in contact with each other when the inner profile is in a clamped position. Other examples of a deflection control profile are possible, however. For example, FIG. 35 illustrates a cross-sectional view of an example of a surgical clamp jaw 686, not forming part of the invention, having a shod 688, wherein the surgical clamp jaw 686 has a deflection control profile 690 defining gaps 692 which are separated by portions of the shod 688. The clamp jaw 686 also has a substantially concave inner profile 694 opposite the deflection control profile 690. As the inner profile 694 is caused to deflect back towards the deflection control profile 690, the shod 688 material in the gaps 692 will be compressed. At some point, depending on the properties of the shod 688 material, the material in the gaps 692 will not compress further under normal clamping forces, and the inner profile will have a second profile shape in the clamped position. In this case, a shod 688 material may be chosen to compress in such a way that this second profile shape (not shown here) is substantially concave, substantially flat, or substantially convex.

In some embodiments, the properties which might make for a good shod material (in terms of gripping ability, for example) might not make for a desired compression property in the gaps defined by the deflection control profile. In such a situation, the shod could include more than one material. For example, FIG. 36 illustrates a cross-sectional view of an example of a surgical clamp jaw 696, not forming part of the invention, having a shod 698 with a first material 700 at least over a portion of the inner profile 702. The shod 698 also has a second material 704 separating the gaps defined by the deformation control profile 706. Alternatively, this second material 704 could be separate from the first material 700 and therefore not part of the shod 698. The second material 704 may be selected for its compression properties independently of the properties of the first material 700. The inner profile 702 is substantially concave in the unclamped position illustrated in FIG. 36. As the inner profile 702 is caused to deflect back towards the deflection control profile 706, the second material 704 in the gaps of the deflection control profile 706 will be compressed. At some point, depending on the properties of the second material 704, the second material 704 will not compress further under normal clamping forces, and the inner profile 702 will have a second profile shape in the clamped position. In this case, the second material 704 may be chosen to compress in such a way that this second profile shape (not shown here) is substantially concave, substantially flat, or substantially convex.

Various advantages of a surgical clamp device and methods for its use have been discussed above. Embodiments discussed herein have been described by way of example in this specification. It will be apparent to those skilled in the art that the forgoing detailed disclosure is intended to be presented by way of example only, and is not limiting. Various alterations, improvements, and modifications will occur and are intended to those skilled in the art, though not expressly stated herein. As just one example, although mechanical knots have been discussed as examples of knotting to hold the first and second clamp jaws in a clamped position, it should be understood that any type of knot or fastening device, including knots tied by hand, forceps, or other manipulator directly into the suture can provide a suitable knotting. Additionally, the recited order of processing elements or sequences, or the use of numbers, letters, or other designations therefore, is not intended to limit the claims to any order, except as may be specified in the claims. Accordingly, the invention is limited only by the following claims.

## Claims

1. A surgical clamp jaw (642, 656), comprising:
an inner profile (644); and
a deflection control profile (646) opposite the inner profile (644);
wherein the deflection control profile (646) comprises one or more sets of corresponding abutment surfaces (652A, 652B, 660A, 660B);
wherein the abutment surfaces (652A, 652B, 660A, 660B) in at least one of the one or more sets of corresponding abutment surfaces (652A, 652B, 660A, 660B) are not contacting each other when the inner profile (644) is in an unclamped position; and
wherein the abutment surfaces (652A, 652B, 660A, 660B) in the at least one of the one or more sets of corresponding abutment surfaces (652A, 652B, 660A, 660B) are in contact with each other when the inner profile (644) is in a clamped position;
**characterized in that** the corresponding abutment surfaces (652A, 652B, 660A, 660B) in the at least one of the one or more sets of corresponding abutment surfaces (652A, 652B, 660A, 660B), comprise:
a first abutment surface (652A, 660A) having a first interlocking feature (654A, 658A), and
a second abutment surface (652B, 660B) having a second interlocking feature (654B, 658b);
wherein the first and second interlocking features (658A, 658B) are not in contact when the inner profile (644) is in the unclamped position; and
wherein the first and second interlocking features (658A, 658B) are configured to interlock when the inner profile (644) is in the clamped position.

2. The surgical clamp jaw of claim 1, **characterized in that**
the inner profile (644) comprises a first substantially concave profile in the unclamped position; and
the inner profile (644) comprises a second substantially concave profile in the clamped position.

3. The surgical clamp jaw of claim 1, **characterized in that**
the inner profile (644) comprises a substantially concave profile in the unclamped position; and
the inner profile (644) comprises a substantially flat profile in the clamped position or
the inner profile (644) comprises a substantially convex profile in the clamped position.

4. The surgical clamp jaw of any one of claims 1 to 3, **characterized in that** the surgical clamp jaw (642) defines one or more flexion assistance voids (632).

5. The surgical clamp jaw of claim 4, **characterized in that** the one or more flexion assistance voids (632) have a sectional shape in a plane substantially perpendicular to the inner profile (644), the sectional shape selected from the group consisting of substantially triangular, substantially circular, substantially rectangular, and substantially oval.

6. The surgical clamp jaw of claim 4 or 5, **characterized in that** at least one of the one or more flexion assistance voids (632) is in contact with a gap (614) between one of the one or more sets of corresponding abutment surfaces (652A, 652B, 660A, 660B).

7. The surgical clamp jaw of any one of claims 1 to 6, **characterized by** a pivot point (648).

8. The surgical clamp jaw of claim 7, **characterized by** an actuator configured to rotate the inner profile (644) of the surgical clamp jaw (642, 656) around the pivot point (648).

9. The surgical clamp jaw of claim 8, **characterized in that** the actuator is selected from the group consisting of a lever, an arm, a gear, a pulley, and a motor.

10. The surgical clamp jaw of any one of claims 1 to 9, **characterized in that** the inner profile (644) comprises a smooth surface.

11. The surgical clamp jaw of any one of claims 1 to 10, **characterized by** a shod (662, 668).

## Patentansprüche

1. Eine chirurgische Klemmbacke (642, 656), umfassend:
ein inneres Profil (644); und ein Auslenkungssteuerprofil (646) gegenüber dem inneren Profil (644);
wobei das Auslenkungssteuerprofil (646) einen oder mehrere Sätze von entsprechenden Anschlagflächen (652A, 652B, 660A, 660B) umfasst;
wobei die Anschlagflächen (652A, 652B, 660A, 660B) in mindestens einem der einen oder mehreren Sätze von entsprechenden Anschlagflächen (652A, 652B, 660A, 660B) einander nicht berühren, wenn das Innenprofil (644) in einer nicht eingespannten Position ist;
und wobei die Anschlagflächen (652A, 652B, 660A, 660B) in dem mindestens einen der einen oder mehreren Sätze von entsprechenden Anschlagflächen (652A, 652B, 660A, 660B) miteinander in Kontakt sind, wenn sich das Innenprofil (644) in einer eingespannten Position befindet;
**dadurch gekennzeichnet, dass** die entsprechenden Anschlagflächen (652A, 652B, 660A, 660B) in dem mindestens einen der einen oder mehreren Sätze von entsprechenden Anschlagflächen (652A, 652B, 660A, 660B) folgendes umfassen:
eine erste Anschlagfläche (652A, 660A) mit einem ersten Verriegelungsmerkmal (654A, 658A), und eine zweite Anschlagfläche (652B, 660B) mit einem zweiten Verriegelungsmerkmal (654B, 658b)
wobei das erste und das zweite Verriegelungsmerkmal (658A, 658B) nicht in Kontakt sind, wenn sich das Innenprofil (644) in der nicht eingespannten Position befindet;
und wobei das erste und das zweite Verriegelungsmerkmal (658A, 658B) so konfiguriert sind, dass sie ineinander greifen, wenn sich das Innenprofil (644) in der eingespannten Position befindet.

2. Chirurgische Klemmbacke nach Anspruch 1, **dadurch gekennzeichnet, dass** das Innenprofil (644) in der nicht eingespannten Position ein erstes, im Wesentlichen konkaves Profil aufweist; und dass das Innenprofil (644) in der eingespannten Position ein zweites, im Wesentlichen konkaves Profil aufweist.

3. Chirurgische Klemmbacke nach Anspruch 1, **dadurch gekennzeichnet, dass** das Innenprofil (644) in der nicht eingespannten Position ein im Wesentlichen konkaves Profil aufweist; und
das Innenprofil (644) in der eingespannten Position ein im Wesentlichen flaches Profil aufweist oder
das Innenprofil (644) in der eingespannten Position ein im Wesentlichen konvexes Profil aufweist.

4. Chirurgische Klemmbacke nach einem der Ansprüche 1 bis 3, **dadurch gekennzeichnet, dass** die chirurgische Klemmbacke (642) einen oder mehrere Flexionsunterstützungshohlräume (632) definiert.

5. Chirurgische Klemmbacke nach Anspruch 4, **dadurch gekennzeichnet, dass** der eine oder die mehreren Flexionsunterstützungshohlräume (632) eine Querschnittsform in einer Ebene aufweisen, die im Wesentlichen senkrecht zu dem Innenprofil (644) verläuft, wobei die Querschnittsform aus der Gruppe gewählt wird, die umfasst: im Wesentlichen dreieckig, im Wesentlichen kreisförmig, im Wesentlichen rechteckig und im Wesentlichen oval.

6. Chirurgische Klemmbacke nach Anspruch 4 oder 5, **dadurch gekennzeichnet, dass** mindestens einer der einen oder mehreren Flexionsunterstützungshohlräume (632) in Kontakt mit einem Spalt (614) zwischen einem der einen oder mehreren Sätzen von entsprechenden Anschlagflächen (652A, 652B, 660A, 660B) steht.

7. Chirurgische Klemmbacke nach einem der Ansprüche 1 bis 6, **gekennzeichnet durch** einen Drehpunkt (648).

8. Chirurgische Klemmbacke nach Anspruch 7, **gekennzeichnet durch** einen Aktuator, der so konfiguriert ist, dass er das Innenprofil (644) der chirurgischen Klemmbacke (642, 656) um den Drehpunkt (648) dreht.

9. Chirurgische Klemmbacke nach Anspruch 8, **dadurch gekennzeichnet, dass** der Aktuator ausgewählt ist aus der Gruppe bestehend aus einem Hebel, einem Arm, einem Getriebe, einer Riemenscheibe und einem Motor.

10. Chirurgische Klemmbacke nach einem der Ansprüche 1 bis 9, **dadurch gekennzeichnet, dass** das Innenprofil (644) eine glatte Oberfläche aufweist.

11. Chirurgische Klemmbacke nach einem der Ansprüche 1 bis 10, **gekennzeichnet durch** einen Beschlag (662, 668).

## Revendications

1. Mâchoire de pince chirurgicale (642, 656), comprenant:
un profil intérieur (644);
et un profil de commande de déviation (646) opposé au profil intérieur (644);
dans lequel le profil de commande de déviation (646) comprend un ou plusieurs ensembles de surfaces de butée correspondantes (652A, 652B, 660A, 660B);
dans lequel les surfaces de butée (652A, 652B, 660A, 660B) dans au moins un des un ou plusieurs ensembles de surfaces de butée correspondantes (652A, 652B, 660A, 660B) ne sont pas en contact les unes avec les autres lorsque le profil intérieur (644) est dans une position non serrée;
et dans lequel les surfaces de butée (652A, 652B, 660A, 660B) dans le au moins un des un ou plusieurs ensembles de surfaces de butée correspondantes (652A, 652B, 660A, 660B) sont en contact les unes avec les autres lorsque le profil intérieur (644) est dans une position serrée;
**caractérisé en ce que** les surfaces de butée correspondantes (652A, 652B, 660A, 660B) dans l'un au moins des un ou plusieurs ensembles de surfaces de butée correspondantes (652A, 652B, 660A, 660B), comprennent une première surface de butée (652A, 660A) ayant une première caractéristique d'interverrouillage (654A, 658A), et une seconde surface de butée (652B, 660B) ayant une seconde caractéristique d'interverrouillage (654B, 658b);
dans lequel les première et seconde caractéristiques d'interverrouillage (658A, 658B) ne sont pas en contact lorsque le profil intérieur (644) est dans la position non serrée;
et dans lequel les première et seconde caractéristiques d'interverrouillage (658A, 658B) sont configurées pour s'interverrouiller lorsque le profil intérieur (644) est dans la position serrée.

2. Mâchoire de pince chirurgicale selon la revendication 1, **caractérisée en ce que** le profil intérieur (644) comprend un premier profil substantiellement concave dans la position non serrée;
et le profil intérieur (644) comprend un second profil substantiellement concave dans la position serrée.

3. Mâchoire de pince chirurgicale selon la revendication 1, **caractérisée en ce que** le profil intérieur (644) comprend un profil substantiellement concave dans la position non serrée; et
le profil intérieur (644) comprend un profil substantiellement plat dans la position serrée ou
le profil intérieur (644) comprend un profil substantiellement convexe dans la position serrée.

4. Mâchoire de pince chirurgicale de l'une quelconque des revendications 1 à 3, **caractérisée en ce que** la mâchoire de pince chirurgicale (642) définit un ou plusieurs vides d'assistance à la flexion (632).

5. Mâchoire de pince chirurgicale selon la revendication 4, **caractérisée en ce qu'**un ou plusieurs vides d'assistance à la flexion (632) ont une forme en coupe dans un plan substantiellement perpendiculaire au profil intérieur (644), la forme en coupe étant choisie dans le groupe constitué par les formes substantiellement triangulaire, substantiellement circulaire, substantiellement rectangulaire et substantiellement ovale.

6. Mâchoire de pince chirurgicale selon la revendication 4 ou 5, **caractérisée en ce qu'**au moins un des un ou plusieurs vides d'assistance à la flexion (632) est en contact avec un espace (614) entre un des un ou plusieurs ensembles de surfaces de butée correspondantes (652A, 652B, 660A, 660B).

7. Mâchoire de pince chirurgicale de l'une quelconque des revendications 1 à 6, **caractérisée par** un point de pivotement (648).

8. Mâchoire de pince chirurgicale selon la revendication 7, **caractérisée par** un actionneur configuré pour faire tourner le profil intérieur (644) de la mâchoire de pince chirurgicale (642, 656) autour du point de pivot (648).

9. Mâchoire de pince chirurgicale selon la revendication 8, **caractérisée en ce que** l'actionneur est choisi dans le groupe constitué par un levier, un bras, un engrenage, une poulie et un moteur.

10. Mâchoire de pince chirurgicale de l'une quelconque des revendications 1 à 9, **caractérisée en ce que** le profil intérieur (644) comprend une surface lisse.

11. Mâchoire de pince chirurgicale de l'une quelconque des revendications 1 à 10, **caractérisée par** un sabot (662, 668).
